# EUROPEAN PATENT SPECIFICATION

(11) **EP 3 860 428 B1**
(45) Date of publication and mention of the grant of the patent: **02.12.2026**
(21) Application number: 19787157.7
(22) Date of filing: 07.10.2019
(51) Int. Cl.: A61B 5/16, A61B 5/00, A61B 5/0205, A61B 5/11, A61B 5/398, A61B 3/113, G16H 40/67, G16H 50/20

(54) **NEUROTRANSMITTER IMBALANCE DETECTION SYSTEM**
SYSTEM ZUR DETEKTION EINES NEUROTRANSMITTER-UNGLEICHGEWICHTS
SYSTÈME DE DÉTECTION DE DÉSÉQUILIBRE DE NEUROTRANSMETTEUR

(30) Priority: 05.10.2018 DK PA201870660
(43) Date of publication of application: 11.08.2021
(73) Proprietor: Dynamic Brain Systems ApS, 8240 Risskov (DK)
(72) Inventor: Bjerrum, Stefan, 8240 Risskov (DK)
(74) Representative: Patentgruppen A/S
(86) International application number: PCT/DK2019/050295
(87) International publication number: WO 2020/069712

(56) References cited:
- US-A1- 2012 196 552
- US-A1- 2012 238 903
- US-A1- 2014 148 728
- US-A1- 2014 357 968
- US-A1- 2015 245 800
- US-A1- 2016 022 137

## Description

### FIELD OF INVENTION

The invention relates to a neurotransmitter imbalance detection system, particularly a system using an eye movement sensor.

### BACKGROUND

Psychiatric disorders typically have very severe consequences for the patient. Not only may psychiatric disorders inflict serious consequences on the patient, but since reliable and objective criteria do often not exist, the diagnosis of psychiatric disorders may in itself be a resource demanding process, which can effectively delay treatment, and also involve risks of misdiagnosis.

Particularly, a simple and reliable test for psychiatric disorders lack, especially when comparing with physical disorders where e.g. a certain pathogen may be identified by tests, sometimes even relatively simple tests.

Some efforts have investigated correlation between certain types of eye movements with certain psychiatric disorders. One example may be seen in Tiancheng W. et al (Closed eye movements in calm state in manic-depressive patients, 1998, Chinese Journal of Psychiatry 1998, 31:2 (97-99)) showed some difference between the eye movements per minute for the manic-depressive patients, schizophrenic patients and healthy persons.

However, no simple and reliable test for psychiatric disorders, such as ADHD have been identified.

Methods for detection of eye movements in relation to Parkinson's disease are known.

US 2015/0245800 A1 to Danmarks Tekniske Universitet relates to a method for detection of an abnormal sleep pattern based on a dataset of Electrooculography (EOG) signals obtained from a sleeping subject. The method may comprise assisting in detection of neurodegenerative disorders such as Parkinson's disease.

US 2012/0238903 A1 to Susana Matinez-Conde et al. relates to methods and apparatus for differential diagnosis of a patient with progressive supranuclear palsy (PSP) from a patient with Parkinson's disease (PD). The method may include identifying a plurality of partially repetitive eye movements.

US 2014/357968 A1 to Chen Lewis relates to measuring neurotransmitter levels, especially dopamine, from reward/non-reward saccadic eye-movement parameters.

### SUMMARY

The invention relates to a neurotransmitter imbalance detection system according to independent claim 1. Preferred embodiments are defined by dependent claims 2-11.

An advantage of the invention may be that it supports and facilitates both identifying psychiatric disorders and treatment of psychiatric disorders. Particularly, this is facilitated by identifying the neurotransmitter imbalance, which the psychiatric disorder is associated with. Thus, effective identification of psychiatric disorders associated with a neurotransmitter imbalance may be provided. In more detail, by identifying certain characteristic eye movement patterns, the neurotransmitter imbalance may be identified in a relatively precise, yet simple and non-invasive manner.

A further advantage of the invention may be that identification of a neurotransmitter imbalance by means of eye movement patterns can be carried out in a relatively simple and cost-effective manner, while obtaining relatively accurate and objective results. Since questionnaires and interviews are often relied upon for diagnostics of psychiatric disorders, subjectivity of the patient and/or the examining practitioner may often pose a risk of leading to inaccurate diagnoses, or may lead to extension of the process of establishing the diagnosis. However, the present invention provides a setup which can provide improving support of such conventional measures, or refer such conventional measures to a secondary potion or even completely replace such.

A further advantage may, as an example, be that treatment may be adjusted and improved by monitoring the difference in output, such as the frequency content signal, e.g. to identify the treatment where a frequency content signal a frequency density indication signal is closest to a normal condition range.

A significant advantage of the invention is that it allows identification of AHDH as a psychiatric disorder by means of a dopamine/noradrenalin imbalance detection. By identifying eye movement patterns characteristic of dopamine/noradrenaline imbalance, the dopamine/noradrenalin imbalance may be identified. Such eye movement patterns may include frequent eye movements, e.g. identified as a high frequency content within the predefined frequency range. A further indicator may be the number of eye movements where the corresponding eye movement signal exceeds a predefined threshold. A further indicator may be the duration in which a person can keep his eye at rest while having closed eyes and refrain from falling asleep. This may be identified as the time period where eye movement signal keeps below a certain predefined threshold.

An advantage of the invention is that it allows identification of dopamine/noradrenalin imbalance by identifying eye movement patterns characteristic thereof. Such eye movement patterns may include frequent eye movements, e.g. identified as a high frequency content within the predefined frequency range.

The present inventor found that deviating values (i.e. when the predetermined threshold is exceeded) of the frequency density indication signal indicated an abnormal condition, where e.g. AHDH subjects could be distinguished well from control subjects. While not providing a diagnosis in itself, the present invention proved effective in improving the accuracy of identification of psychiatric disorders, such as ADHD and other disorders as described herein.

The present invention may advantageously be used to evaluate treatment by medication. Lowering the frequency density, e.g. represented by a maximum of a frequency distribution, indicates an effective treatment of e.g. ADHD, whereas increasing the frequency density, e.g. represented by a maximum of a frequency distribution, indicates a non-effective treatment.

The present invention may advantageously be used to identify subjects that has an increased risk of developing addiction to a medication. By obtaining a frequency density indication signal before and after intake of the medication, having a positive effect of the medication combined with an increase in the frequency density indication signal indicates risk of developing addiction to the mediation.

The present invention may advantageously be used to detect current substance abuse of a medication. By obtaining a frequency density indication signal before and after intake of the medication of a person normally using the medication but who is currently taking any medication (e.g. 24-48 hours before the test), it can be evaluated if an increased frequency density indication signal indicates risk of substance abuse of the medication, or a decreased frequency density indication signal indicates no abuse. This may also be used when testing for substance abuse among subjects with chronic pain disorders. While it may be difficult for a medical practitioner to establish whether a patient still experiences pain or if a substance addiction has been evaluated, it is believed that the present invention may facilitate screening by looking at whether the frequency density indication signal is increased or decreased when administering the medication in question. Similarly, the test may be applied before startup of the treatment of a certain medication to evaluated if the subject has an increased risk of developing substance abuse.

Further, the present invention may advantageously be used to evaluate whether the applied dosage of the medication is sufficient or should be increased. By testing the evolution of the frequency density indication signal before and after administering the mediation (i.e. e.g. 2-3 hours after the medication has been ingested and should be effective) and at further points in time. If the desired effect on the frequency density indication signal is insufficient, the dosage of the medication may be adjusted accordingly.

The frequency density may also be referred to as power density, i.e. the density of frequency components for the frequency range in question.

The present invention may advantageously benefit from the frequency analysis arrangement being computer implemented. Thereby, the at least one eye movement signal is automatically communicated to said frequency analysis arrangement, where it is automatically received and processed, where the frequency density indication signal is automatically outputted, and where the frequency analysis arrangement automatically determines if said frequency density indication signal exceeds a predetermined threshold. Thus, the operator may typically initiate measurements, after which the operation and processing is automatic until it is been determined if the predetermined threshold is exceeded. In various embodiments, it is communicated to the operator if the predetermined threshold is exceeded or not, possibly also giving a value of the frequency density indication signal.

In embodiments of the invention, different parameters may be used as the frequency density indication signal correlating with a frequency density. Important parameters may be, according to embodiments of the invention, maximum value of the frequency distribution, sum of frequency, average frequency density in the predefined frequency range, and combinations thereof. Average frequency densities include different types of averages, such as normal average, root mean square (RMS) etc. Values correlating with such average values may also be used, e.g. the product of the average amplitude within a predefined frequency range and the weighted frequency average within the predefined frequency range. Thus, a frequency content signal is one example of a frequency density indication signal.

In embodiments of the invention, a medical practitioner may use the outputted signal as a diagnostically relevant input, thus aiding the process of diagnosing by replacing less reliable measures and/or by providing a further input of diagnostic relevance having a high reliability and being objective in the sense that it does not involve a subjective input from the patient or the practitioner. In other words, the methods are envisioned to be used in conjunction with other tools selected by the health care person or medical practitioner.

In embodiments of the invention, an output signal representing the frequency content in the predefined frequency range may be provided as a numerical value, as a set of numerical values, such as frequency component coefficients, or as a representation thereof. When the signal is provided as a representation, such as a graphical representation, this may be as e.g. a curve or a diagram, e.g. a bar chart. Graphical representations are particularly suitable for providing sets of numerical values. Alternatively, the signal may be provided as a signal representing a number of predefined outcome scenarios. For example, the signal may represent a positive or negative result of a comparison with an abnormal range, such that a positive represents that the patient displays abnormal eye movement patterns whereas a negative represents that the patient displays normal eye movement patterns, or vice versa. This may be indicated in a number of different ways, e.g. by numbers, values, codes, letters, colors, symbols, combinations thereof, etc. Further predefined outcome scenarios may also be included, such as graduated normality (normal, slightly elevated level of significant eye movement patterns, significantly elevated level of significant eye movement patterns, etc.).

Without being bound theory, it is believed that many psychiatric disorders are related to a neurotransmitter imbalance. For example, it is believed that ADHD (Attention Deficit Hyperactivity Disorder) is related to an imbalance in the dopamine noradrenaline neurotransmitter balance. Such neurotransmitter imbalances are furthermore believed to be detectable by means of eye movement observations. Since eye movements are dominated by visual impressions received from surroundings, even when these are relatively calm, and the thoughts resulting thereof, it is believed that observation of closed eyes are necessary and beneficial to receive observations, which are not influenced by such visual impressions. Specifically, it is believed that the intrinsic unrest associated with ADHD leads to an increased activity and different eye movement pattern when having closed eyes. By detecting this increased activity and different eye movement pattern it is believed that a parameter of significant diagnostic relevance may be obtained.

Other psychiatric disorders may also lead to a neurotransmitter imbalance, again resulting in different eye movement patterns. For example, schizophrenia, anxiety, depression, Obsessive-compulsive Disorder (OCD), personality disorders, Attention Deficit Disorder (ADD), bipolar disorder, Post Traumatic Stress Disorder (PTSD), etc. Movement in other frequency ranges may be of particular interest for these conditions.

As used herein the term "neurotransmitter imbalance detection system" is a system configured to output parameters or signals from which a neurotransmitter imbalance can be identified. It should be mentioned that in some embodiments, at least one further parameter may be used to assist in the identification of the neurotransmitter imbalance. Such further parameter(s) may be provided by the detection system, or by other means, e.g. when such further parameter(s) comprises e.g. blood pressure, heart rate etc.

As used herein the term "eye movement sensor" refers to a sensor configured to sense eye movements in a closed eye. It is noted that while some eye sensors may be used both for sensing eye movements of a closed eye and an open eye, others may be specifically designed to sense eye movements in closed eyes. Moreover, the sensor is adapted to out an indication of an eye movement in the form of an eye movement signal. In some embodiments, the deviation from a constant level is proportional to a change in position relative to an initial position or resting position, whereas in other embodiments the relationship between the signal level and an exact position of the eyes is more complex and the exact eye position may not necessarily be established from the eye movement signal. Even in some embodiments, having eyes in complete resting does not translate into a constant level signal. Nevertheless, using frequency analysis, distinct eye movement patterns may be identified, which can be correlated to a normal range or an abnormal range, whereby a neurotransmitter imbalance may be detected.

As used herein the term "frequency analysis arrangement" refers to an arrangement comprising one or more devices which together and automatically execute frequency analysis, such that the frequency content signal is established from the eye movement signal. An important aspect of the frequency analysis arrangement is that it comprises software implemented in hardware, i.e. one or more devices comprising memory and a processing unit. The hardware may be local and even physically connected to the eye movement sensors by electronic wirings or remotely positioned and communicate with the eye movement sensors at least partly through the internet. The frequency analysis is thus performed by automatic execution of hardware implemented software when receiving the eye movement signal.

As used herein the term "eye movement signal" is a signal representing eye movements over a given time period. The signal may e.g. be transmitted as an electronic signal, either digitally or analogue, or an optical signal.

As used herein the term "frequency analysis" refers to the operation of converting a time signal into frequency space, i.e. establishing the distribution of power into frequency components composing the original time series signal. Various algorithms may be employed, hereunder for example Fourier transformation-based algorithms, such as Fast Fourier Transformation (FFT) based algorithms, etc.

As used herein the term "frequency distribution" refers to the relative distribution of frequency. Typically, using discrete recorded signals, the frequency distribution refers to the distribution of frequency components within a certain predefined frequency range. Thus, processing a signal by frequency analysis to determine a frequency distribution refers to the process of establishing the signal in frequency space, which typically may be to establish the value of each frequency component within the predefined frequency range.

As used herein the term "frequency content signal" refers to signal illustrating the distribution between different frequency components. According to various embodiments of the invention, the displaying of the frequency content signal may be done via many different platforms, such as for example same device, a personal computer, a tablet, a phone etc. The frequency content signal may be displayed as a numerical value, or an array of numerical values, or be visualized in different ways, for example as a curve, histogram etc.

As used herein the term "predefined frequency range" refers to a range of frequencies considered relevant for identifying a neurotransmitter imbalance. Particularly, the frequency content within this range and optionally distribution of within the range is considered important. The frequency range is predefined in the sense that the frequency analysis arrangement is configured to output the frequency content signal for this particular frequency range. Typically, the frequency range may differ, depending on the particular neurotransmitter imbalance(s) the system is configured to detect. The predefined frequency range is a range, i.e. more than just one value, and includes at least the range of 1 to 3 Hz, such as 1 to 5 Hz. In an embodiment the predetermined frequency range is 1 to 5 Hz.

As used herein the term "relative content" refers to e.g. the relative content in the predefined frequency range, signifying the ratio between the total frequency content within the predefined frequency range and the total frequency content obtained from the frequency analysis.

As used herein the term "normal range representation" refers to a representation associated with a normal condition, i.e. absence of a neurotransmitter imbalance.

As used herein the term "abnormal range representation" refers to a representation associated with an abnormal condition, i.e. presence of a neurotransmitter imbalance. As used herein the terms "patient", "person", and "subject" is used somewhat interchangeably to refer to human individuals. Typically, patient may refer to an individual having a diagnosis, whereas person and subject is used more broadly.

According to an embodiment, the frequency content signal is usable as an indication of whether a neurotransmitter imbalance is present or not. Thus, it can be concluded from the frequency content signal, e.g. by suitable comparison with reference values or reference measurements if a neurotransmitter imbalance is present or not. In some alternative embodiments, it may be desirable to include further parameters when identifying a neurotransmitter imbalance, e.g. to improve accuracy.

According to an advantageous embodiment the frequency analysis arrangement is further configured to output a frequency content signal representing a frequency content within a predefined frequency range.

The frequency content in said predefined frequency range may also be seen as the area under the part of the frequency distribution curve corresponding to said predefined frequency range. The frequency content signal is an example of the frequency density indication signal.

An advantage of the above embodiment may be that an accurate determination of a diagnosis is facilitated.

One advantage of the above embodiment may be that discerning attention deficit hyperactivity disorder (ADHD) from attention deficit disorder (ADD).

According to an advantageous embodiment the system further comprises an oscillation analysis arrangement, the oscillation analysis arrangement, the oscillation analysis arrangement being configure to
receive said at least one eye movement signal, and
process said at least one eye movement signal.

The oscillation analysis arrangement is configured to analyze the eye movement signal and output a value in response thereto. Different types of output values may be used, as illustrated by the below embodiments. The oscillation analysis may be or include a statistical analysis of the signal in the time domain with respect to occurrence or time distribution of its amplitude values (or zero-crossings), or by integrating the signal over a determined time period (i.e. determining the sum of the signal).

In embodiments, said oscillation analysis arrangement is computer implemented and its operation is automatic.

According to an advantageous embodiment the oscillation analysis arrangement is configured to
process said at least one eye movement signal to determine the occurrence rate of oscillations exceeding a predetermined threshold.

In one embodiment, the subjects are categorized according to the occurrence rate of large eye movement, i.e. eye movements where the oscillation amplitude of the eye movement signal exceeds a predetermined threshold. For example, this may be used to see if such large eye movements are present almost all the time, or if pauses occur between these. As an illustrative example, the subjects may be categorized into e.g. three groups where the eye movement signals recorded over a predetermined time period displays, where the first group is associated with no pause exceeding 5 seconds between large eye movements, the second group is associated with at least one pause between large eye movements above 5 seconds, but none above 20 seconds, and the third group being associated with at least one pause between large eye movements above 20 seconds.

According to an advantageous embodiment the oscillation analysis arrangement is configured to
process said at least one eye movement signal by to determine a representative amplitude of eye movements associated with an oscillation of the eye movement signal exceeding a predetermined threshold.

According to embodiments, the representative amplitude may be determined in various ways. For example, when the eye movements associated with an oscillation of the eye movement signal exceeding a predetermined threshold, also referred to as large eye movements, have been identified, the representative amplitude may be determined as the average amplitude of the associated parts of the eye movement signal, or as other representative values, such as e.g. the median value or as the one of the largest values (e.g. the sixth highest value).

According to an embodiment, a so-called large eye movement is an eye movement associated with an oscillation of the eye movement signal exceeding a predetermined threshold, which predetermined threshold may for example be half of a maximum oscillation, e.g. when the subject is instructed to move his/her eyes as much as possible.

It is noted that the oscillation analysis arrangement may be configured to perform different types analysis according to embodiments of the invention. In some embodiments, the oscillation analysis arrangement may be integrated in the frequency analysis arrangement.

According to an advantageous embodiment the oscillation analysis arrangement is configured to
process said at least one eye movement signal by to determine the occurrence rate of zero crossings of the eye movement signal.

In the present context, the term "zero crossings" is understood as having is usual meaning, i.e. the events where the signal in question crosses the zero line on a usual graphic representation.

According to an advantageous embodiment the oscillation analysis arrangement is configured to
process said at least one eye movement signal by to determine the total sum of the eye movement signal.

In other words, the values constituting the eye movement signal are summed together to obtain a single value, the sum of the eye movement signal.

According to an advantageous embodiment the system is further configured to determine the maximum value of the frequency distribution.

The maximum value of the frequency distribution may also be referred to as the amplitude of the frequency distribution. Thus, when e.g. a Fast Fourier transform algorithm is used to obtain the frequency distribution, the maximum value of the frequency distribution is the maximum value of the obtained FFT-curve. Thus, the maximum value of the frequency distribution may be determined by the frequency analysis arrangement.

The maximum value of the frequency distribution is an example of the frequency density indication signal.

According to an advantageous embodiment the system is further configured to determine the maximum value of the frequency distribution within the predefined frequency range.

According to an advantageous embodiment said frequency analysis arrangement is further configured to compare said frequency content signal with a normal range representation and an abnormal range representation.

According to an advantageous embodiment of the invention said frequency analysis arrangement is further configured to determine if relative content in said predefined frequency range exceeds a predetermined threshold.

According to an embodiment, the predetermined threshold is at least 10% of the total frequency content, such as at least 20% of the total frequency content, such as at least 30% of the total frequency content, such as at least 40% of the total frequency content, such as at least 50% of the total frequency content, such as at least 60% of the total frequency content, such as at least 70% of the total frequency content, such as at least 80% of the total frequency content.

The relative content in a predefined frequency range may also be seen as the area under the part of the frequency distribution curve corresponding to said predefined frequency range divided by the total area under the frequency distribution curve.

According to an advantageous embodiment the frequency analysis arrangement comprises at least one digital memory, wherein the at least one digital memory comprises a normal range representation and an abnormal range representation.

According to an advantageous embodiment the frequency analysis arrangement comprises at least one digital memory, wherein the frequency analysis arrangement is configured to store at least the frequency content signal.

One advantage of the above embodiment may be that an outputted frequency content signal and optionally also further parameters and/or raw signals, such as the eye movement signal, can be compared, manually and/or automatically, with earlier recorded values or signals. Thereby, it may be possible to identify and track differences e.g. related to initiation and/or adjustment of medication, whereby the medication of the individual subject may be improved and optimized.

According to an advantageous embodiment the frequency content signal represents a value, such as a numerical value.

In an embodiment of the invention, the frequency content signal is a numerical value representing the frequency content within a predefined frequency range, e.g. obtained by integrating the frequency analysis output over the predefined frequency range.

According to an advantageous embodiment the frequency content signal represents a set of values comprising at least two values, such as at least two numerical values.

According to an advantageous embodiment the frequency content signal further represents the distribution of the frequency content within the predefined frequency range.

According to an advantageous embodiment the neurotransmitter imbalance detection system further comprises a display arranged to display a representation of the frequency content signal.

According to an advantageous embodiment the predefined frequency range comprises at least the range from 1 to 3 Hz, preferably from 1 Hz to 5 Hz.

The predefined frequency range may be used in a number of other embodiments, e.g. for determining where an indication of a frequency content is determined, where a representation of a frequency content is determined, and where a relative content is compared with a predetermined threshold.

According to an embodiment the predefined frequency range comprises at least the range from 1 Hz to 5 Hz.

According to an embodiment of the invention the predefined frequency range comprises at least the range from 1 Hz to 10 Hz.

According to an advantageous embodiment the predefined frequency range comprises at least the range from 0.1 to 3 Hz, preferably from 0.1 Hz to 5 Hz.

According to an advantageous embodiment the frequency analysis is performed by a Fast Fourier Transformation (FFT) based algorithm.

According to an advantageous embodiment the system is further configured to identify at least one further auxiliary characteristic, such as a heart rate, breathing characteristic, or a skin surface tension.

According to an advantageous embodiment the system further comprises an analysis unit configured to identify at least one further characteristic of eye movements.

One advantage of the above embodiment is that it facilitates a more accurate identification of neurotransmitter imbalance. Using the at least one further characteristic of the eye movement together with the determined frequency content facilitates distinguishing between subjects having a neurotransmitter imbalance and other subjects, which display an eye movement pattern otherwise characteristic for neurotransmitter imbalance, but for other reasons, such as e.g. momentarily increased levels of e.g. adrenaline.

According to an advantageous embodiment the at least one further characteristic of eye movements comprises the longest period within which a person, having closed eyes, can abstain from moving the eyes.

One advantage of the above embodiment is that it facilitates a more accurate identification of neurotransmitter imbalance. Particularly, it facilitates distinguishing between subjects having a neurotransmitter imbalance and other subjects, which display an eye movement pattern otherwise characteristic for neurotransmitter imbalance, but for other reasons, such as e.g. momentarily increased levels of e.g. adrenaline. Thus, in other words, the above embodiment is directed to identify a period of no movements. In practice, a threshold for eye movement may be set, such that a measured characteristic or a characteristic derived therefrom must cross the threshold to qualify as an eye movement and also distinguish from e.g. noise.

The longest period within which a person, having closed eyes, can abstain from moving the eyes may also be referred to as the latency time or the latency period.

In an aspect of the invention, the above embodiment involves the provision that the limitation of claim 1 is not adhered to.

According to an advantageous embodiment the system further comprising an analysis unit configured to identify the number of eye movement events within a predefined time range.

One advantage of the above embodiment is that it facilitates a more accurate identification of neurotransmitter imbalance.

According to an advantageous embodiment the eye movement events fulfill one or more selection criteria, such as the eye movement signal displaying an amplitude exceeding a predefined threshold.

One advantage of the above embodiment is that it facilitates a more accurate identification of neurotransmitter imbalance.

According to an advantageous embodiment the system further comprises a breathing sensor.

According to an advantageous embodiment the system further comprises a pulse sensor.

According to an advantageous embodiment the system further comprises a skin surface tension sensor.

According to an advantageous embodiment the system comprises at least two eye movement sensors.

According to an advantageous embodiment the at least one eye movement sensor comprises a tension sensor, such as a piezoelectric sensor.

A tension sensor may typically be positioned e.g. centrally on the eye, and may be configured to detect muscle tension giving a measure related to eye movements.

According to an advantageous embodiment the at least one eye movement sensor comprises a movement sensor, such as an accelerometer or a gyroscope-based sensor, configured to detect movement.

Such a sensor is described e.g. in international application WO 2014/053534.

According to an advantageous embodiment the at least one eye movement sensor comprises a strain gauge sensor.

According to an advantageous embodiment the at least one eye movement sensor comprises an electrooculographic sensor.

According to an advantageous embodiment the at least one eye movement sensor comprises a laser sensor. For example, the sensor may be configured to detect changes in reflections of a laser beam emitted by the laser sensor. The laser beam may, as an example, be reflected by a suitable plate or object positioned on the closed eye lid, such that movements of the eye below the eye lid leads to a different position and/or orientation of the plate or object positioned on the eye lid, again leading to a detectable change in the reflections of the laser beam.

According to an advantageous embodiment the at least one eye movement sensor comprises a magnetic sensor.

For example, a stationery magnetic sensor may detect changes in magnetic field from a magnet affixed to the eye lid, or vice versa.

According to an advantageous embodiment the at least one eye movement sensor comprises a liquid sensor. For example, the liquid sensor may be formed as a container with liquid where the container has a flexible side (e.g. membrane) in abutment with the eye lid. When the eye is moved, the movements lead to movements of the liquid in the container due to the flexible side, and such movements may be detected e.g. by pressure sensors or other suitable sensors.

According to a further embodiment , the at least one eye movement sensor comprises a camera-based eye movement sensor configure to detect eye movements of a closed eye.

According to an advantageous embodiment the system further comprises a communication arrangement, said communication arrangement being arranged to communicate data between said eye movement sensor and said frequency analysis arrangement.

According to an advantageous embodiment said eye movement sensor and said frequency analysis arrangement are configured to communicate via the internet.

The system may also be regarded as a system for detecting an eye movement pattern provided that it is configured to output a signal corresponding to a frequency range relevant for assessment of a neurotransmitter imbalance.

According to an advantageous embodiment the neurotransmitter imbalance is dopamine/noradrenaline and/or is associated with Attention Deficit Hyperactivity Disorder (ADHD).

According to an advantageous embodiment the system further is further configured to correlate at least two selected from said frequency content signal, said occurrence rate of oscillations exceeding a predetermined threshold, said representative amplitude of the eye movement signal, said occurrence rate of zero crossings, and said total sum of the eye movement signal.

An advantage of the above embodiment is that a more accurate result with respect to an estimated diagnosis may be obtained. Non-limiting examples of suitable correlations include e.g. the total sum of the eye movement signal correlated with the occurrence range of zero crossovers, the representative amplitude of the eye movement signal correlated with the occurrence range of zero crossovers, and the representative amplitude of the eye movement signal correlated with the maximum value of the frequency distribution.

According to an advantageous embodiment the system is configured to output an evaluation value indicating a degree of positive response to a medication, the evaluation value being based on at least
- a first eye movement signal recorded prior to intake of the medication and
- a second eye movement signal recorded after intake of the medication.

According to an advantageous embodiment the medication is methylphenidate.

In an embodiment, said frequency analysis arrangement is implemented in a computer system comprising a processor for automatically processing said at least one eye movement signal by frequency analysis to determine said frequency distribution.

In an embodiment, said oscillation analysis arrangement is implemented in said computer system for automatically processing said at least one eye movement signal.

### FIGURES

The invention will now be described with reference to the figures where
Figures 1A-1B illustrate neurotransmitter imbalance detection systems according to embodiments,
Figure 2A-2C illustrate eye movements sensors according to an embodiment,
Figure 3 illustrates an eye movement signal according to an embodiment,
Figure 4A-4F illustrate frequency analyzed eye movement signals of 5 minutes according to embodiments,
Figure 5A-5D illustrate frequency analyzed eye movement signals of 60 seconds according to embodiments,
Figure 6A-6D illustrate frequency analyzed eye movement signals before and after treatment according to embodiments, and
Figure 7A-B illustrate a computer system arranged to implement the neurotransmitter imbalance detection system according to embodiments.

### DETAILED DESCRIPTION

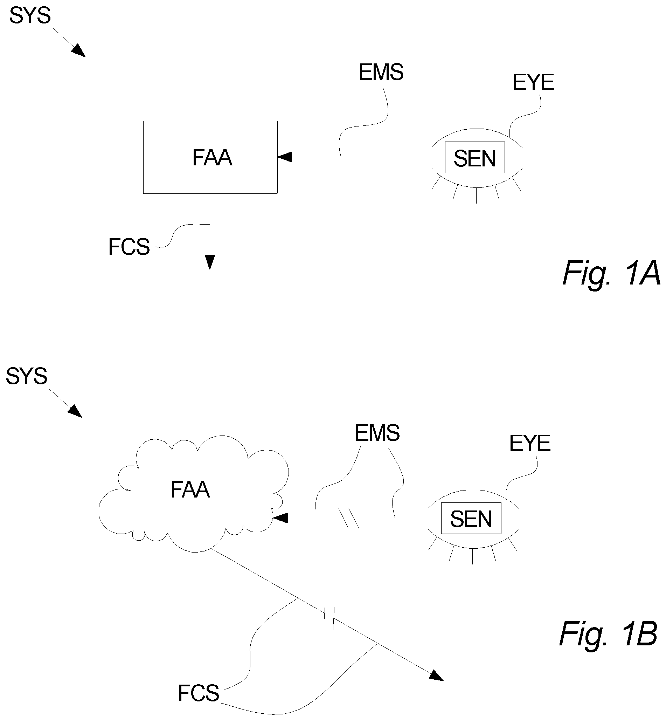

Referring to figure 1A, a neurotransmitter imbalance detection system SYS according to an embodiment is illustrated.

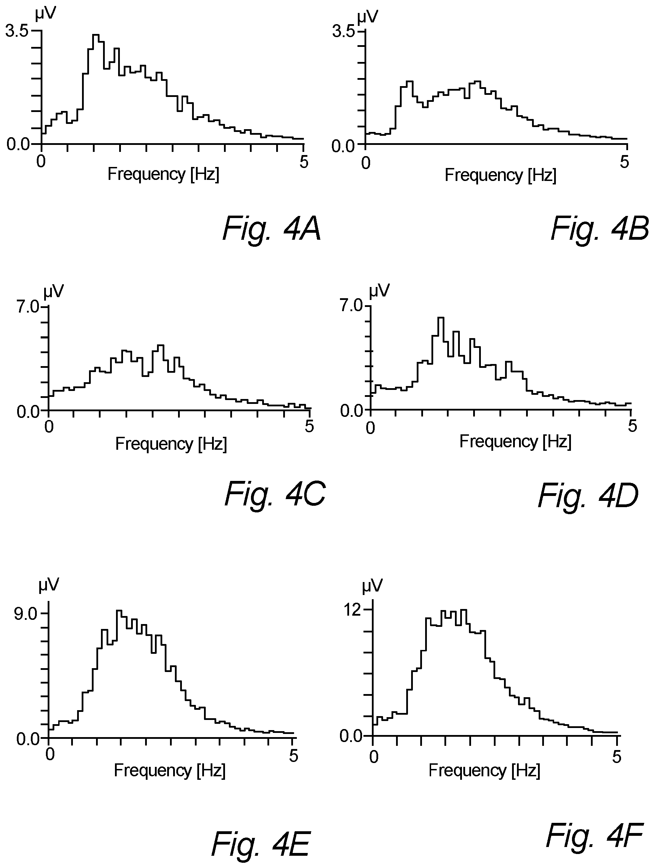

The neurotransmitter imbalance detection system SYS comprises at least one eye movement sensor SEN and a frequency analysis arrangement FAA. The eye movement sensor SEN is adapted to sense movement of a closed eye EYE. During operation, the eye movement sensor SEN is fitted one the subject so as to sense eye movements in the subject, while the subject has closed eyes EYE. The subject is instructed to keep awake during the test. Various types of eye movement sensors SEN are usable within the embodiment of figure 1A, e.g. the eye movement sensors SEN illustrated on figure 4.

The frequency analysis arrangement FAA may for example be a dedicated processing unit connected to the eye movement sensor SEN, or a personal computer to which the eye movement sensor SEN is connected. In any case, the frequency analysis arrangement FAA must be configured to perform frequency analyses.

As seen in figure 1A, the eye movement sensor SEN is configured to output at least one eye movement signal EMS, which is then communicated to the frequency analysis arrangement FAA. The at least one eye movement signal EMS represents movement of the closed eye EYE, typically such that a constant or near-constant level signal corresponds to no or only insignificant eye movements, whereas large deviations from the constant level signifies larger eye movements.

The frequency analysis arrangement FAA is configured to receive said at least one eye movement signal EMS and thereafter process the at least one eye movement signal EMS by frequency analysis to determine a frequency distribution, i.e. a frequency distribution of the eye movement signal EMS. Various methods of frequency analysis may be employed, e.g. a Fast Fourier Transformation (FFT) based algorithm.

Finally, the frequency analysis arrangement FAA outputs a frequency content signal FCS representing the frequency content within a predefined frequency range. The output may be presented to an operator in a wide range of different manners, e.g. as a diagram showing frequency components within at least the predefined frequency range, or as a sum of frequency components within the predefined frequency range. The output may in some embodiments be displayed on a dedicated screen, whereas in other embodiments it may be transmitted to the operator via the internet, e.g. to a computer program linked to the eye movement sensor SEN or even transmitted to the operator via by a digital message, such as an email.

In some further embodiments, the frequency analysis arrangement FAA is arranged to operate in accordance with figure 1A, with the exception that as an alternative to the frequency content signal FCS or in addition thereto, frequency analysis arrangement FAA is configured to output a different signal of diagnostic relevance.

Referring to figure 1B, a neurotransmitter imbalance detection system SYS according to an embodiment of the invention is illustrated. This embodiment is similar to the embodiment of figure 1A, whereas in the embodiment figure 1B, the frequency analysis arrangement FAA is cloud based, i.e. provided at a remote location, and where the eye movement signal EMS is transmitted e.g. via the internet. The eye movement sensor SEN may then be connected directly to the internet, either by cabled connection or by wireless connection, or may be connected to an internet connected device, such as a personal computer, facilitating the connection to the cloud-based frequency analysis arrangement FAA.

Non-limiting examples of important parameters include the following
- analysis of data in time domain, for example to obtain total sum of all values of the eye movement signal, average amplitude of oscillations of the eye movement signal, average frequency of the eye movement signal, occurrence rate of zero crossings of the eye movement signal, etc.,
- frequency analysis, for example to obtain frequency content in certain frequency ranges, e.g. dominating frequency ranges measured by e.g. amplitude size,
- occurrence rate of large eye movements, e.g. eye movements correlated with an eye movement signal exceeding a certain threshold, which large eye movements may be indicative of too high activity in the brain,
- latency time, corresponding the longest period within which a person, having closed eyes, can abstain from moving the eyes, and which period is indicative of whether the person in question is in a balanced state of mind,
- velocity of large eye movements, giving a measure of how fast and how powerful the eye muscles are activated,
- the individual eye movements, and whether they are slow eye movements or rapid eye movements, and whether they are smooth and uniform eye movements, and the degree of variations between individual the eye movements.

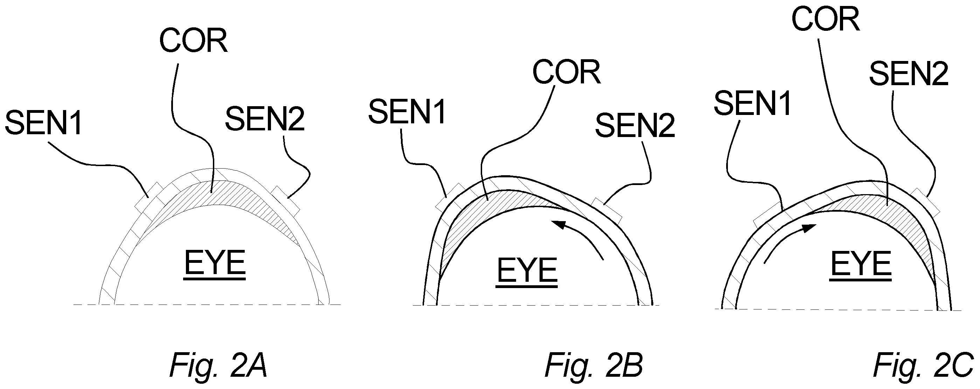

Referring to figures 2A-C, recording to eye movement signals EMS 1, EMS2 by means of two eye movement sensors SEN1, SEN2 is illustrated in accordance with an embodiment.

In figure 2A, the cornea COR is positioned centrally looking, whereas the cornea COR is facing towards the left in figure 2B and right in figure 2C.

The eye movement sensors SEN1, SEN2 may for example be motion sensors, such as a gyroscope-based sensor or an accelerometer based sensor. When comparing the eye movement signals EMS 1, EMS2 from the sensors, a measure for the movement of the eye may be obtained. In this embodiment, the measure for the movement may be obtained by subtracting the two eye movement signals EMS 1, EMS2 to obtain a differential signal. This differential signal may signify movement along a lateral direction defined by an axis between the two eye movement sensors SEN1, SEN2.

In an alternative embodiment, only a single eye movement sensor SEN may be employed. In such embodiments, the eye movement signal EMS may not necessarily allow to distinguish between eye movements if different directions. Nevertheless, the eye movement signal EMS may still contain information about the frequency, speed, and intensity of the eye movements.

Still in further alternative embodiments, the system SYS may have three or more eye movement sensors SEN. Having an increased number of eye movement sensors SEN may help to increase resolution of eye movements to form a more accurate measure of the eye movements. Also, it may help to distinguish e.g. lateral eye movements from vertical eye movements.

Returning to figure 2A-C, it can be observed that when the cornea COR moves towards the left (figure 2B), the eye movement sensor SEN1 is affected and moves away from the eye, whereas the eye movement sensor SEN2 is relatively unaffected. Similarly, when the eye moves towards the right (figure 2), the opposite can be observed, i.e. the sensor SEN1 is affected whereas the sensor SEN2 is relatively unaffected.

When the eye movement is only slight, i.e. the cornea moves only a few degrees in angular position, only one of the sensors SEN1, SEN2 may typically be affected. Also, the time between the oscillations in the signal EMS 1 to a corresponding oscillation in the signal EMS2 signifies the speed with which the eye EYE moves. E.g. longer times suggests a slow movement. Furthermore, when e.g. an accelerometer-based eye movement sensor, a high-speed eye movement results in a faster acceleration of the sensor SEN1, SEN2, again resulting in a higher signal.

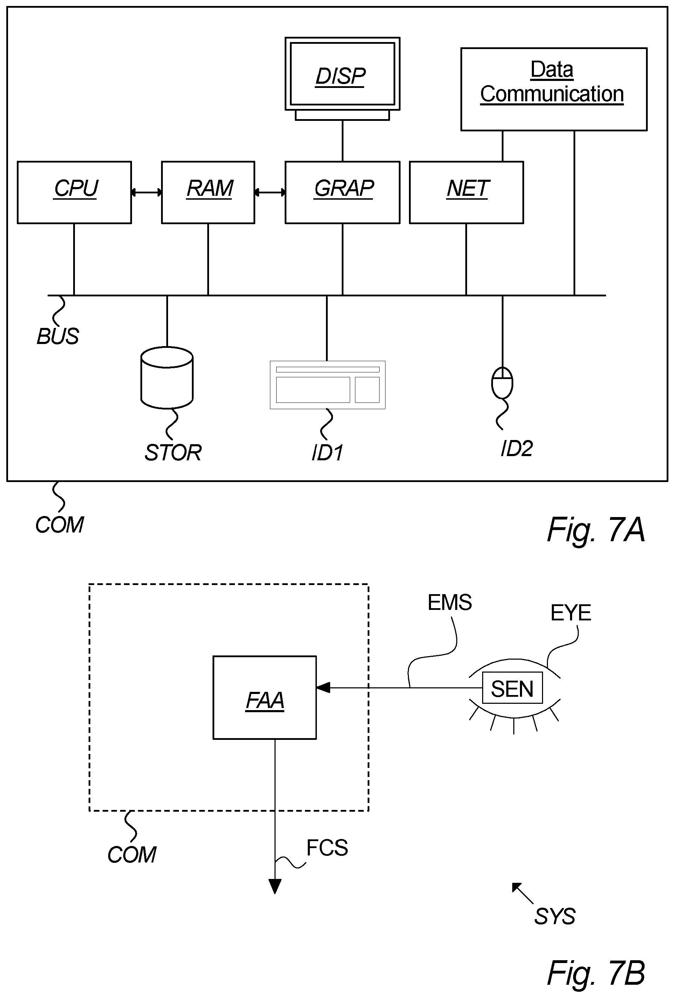

Fig. 7A illustrates the schematics of a computer system COM capable of implementing the neurotransmitter imbalance detection system according to embodiments of the invention. The computer system COM shown in fig. 7A is a stand-alone computer system, however in other embodiments of the invention, the computer system COM may be a distributed computer system, such as a cloud-based computer system in which the computer system shown in fig. 7A represents a node of the cloud-based computer system. The computer system COM comprises a processor CPU coupled to a bus. Also coupled to the bus are a memory RAM, a storage device STOR, a first input device ID1 such as a keyboard, a graphics adapter GRAP, a second input device ID2 such as a pointing device, and a network adapter NET. A display DISP is coupled to the graphics adapter GRAP. The processor CPU may be any general-purpose processor capable of executing computer implemented instructions. The storage device STOR may be any device capable of holding large amounts of data, like a hard drive, solid state drive, compact disk-read-only memory (CD-ROM), DVD, Blu-Ray, or some form of removable storage device. The memory RAM is arranged to hold instructions and data to be used by the processor. The second input device ID2 may be a computer mouse, track-ball, light pen, touch-sensitive display, or other type of pointing device. The first input device ID1 may be a QWERTY keyboard and may be a physical computer keyboard or a keyboard implemented in a touch-sensitive display, such as the same touch-sensitive display implementing the pointing device. The input devices may be used by a user of the computer system, such as an operator, for automatic execution of commands specified by the user. The network adapter NET couples the computer system COM to a network, such as a distributed network, e.g. the internet. The network adapter may also establish a connection between the computer system COM and external devices by means of wireless connection protocols such as WiFi, Bluetooth and Zigbee.

The computer system COM comprises is arranged to establish a data communication DATA between the computer system COM and external devices such as sensors, e.g. one or more eye movement sensors SEN (not shown). The data communication DATA may be a wireless data communication implemented by the use of the network adapter, such as a Bluetooth connection, or a wired data communication implemented by a wired connection to the computer system COM, such as by a wired Universal serial bus (USB) connection. For example, the data communication DATA may facilitate transmittal of eye movement signals EMS from eye movement sensors SEN to the computer system COM.

The computer system COM may be arranged to connect with external sensors such as an eye movement sensor SEN, a breathing sensor, a pulse sensor, a skin surface tension sensor, a tension sensor, such as a piezoelectric sensor, a movement sensor, such as an accelerometer or a gyroscope-based sensor configured to detect movement, a strain gauge sensor, an electrooculographic sensor, a laser sensor, a magnetic sensor, and a liquid sensor.

The computer system COM may represent a desktop computer, a laptop computer or any other electronic device including means of processing such as a tablet computer, a smartphone and a smartwatch in which the input devices ID1-ID2 may be the same touch screen as the display DISP.

The computer system COM may be arranged to implement a frequency analysis arrangement FAA according to embodiments of the invention.

The computer system COM may be further arranged to implement an oscillation analysis arrangement according to embodiments of the invention.

Fig. 7B illustrates a computer implemented neurotransmitter imbalance detection system SYS comprising a frequency analysis arrangement FAA according to embodiments .

The figure illustrates that the frequency analysis arrangement FAA is implemented in a computer system COM, such as the computer system shown in fig. 7A.The computer system COM is arranged to receive an eye movement signal EMS, representing movement of the closed eye (EYE) provided by an eye movement sensor SEN according to various embodiments of the invention, and output a frequency content signal FCS. The output may be displayed to an operator on a display (not shown) of the computer system COM, or it may be transmitted to the operator via the internet, e.g. to a computer implemented program linked to the eye movement sensor SEN or even transmitted to the operator via by a digital message, such as an email. Such wireless transmission may be facilitated by the network adapter of the computer system COM.

The steps of processing eye movement signals EMS by frequency analysis to determine a frequency distribution is performed automatically by means of the frequency analysis arrangement FAA being implemented in a computer system COM. In this sense, the steps of processing eye movement signals is carried out by the processor of the computer system COM. In the case where the computer system COM is a distributed computer system, such as a cloud-based computer system, the steps of processing eye movement signals may be performed by one or nodes (processors) of the distributed computer system.

The invention is solely defined by the scope of the appended claims.

### FIGURE REFERENCES

SYS. Neurotransmitter imbalance detection system
SEN. Eye movement sensor
EYE. Eye
EMS. Eye movement signal
FAA. Frequency analysis arrangement
FCS. Frequency content signal
COR. Cornea
COM. Computer system
CPU. Processor
DATA. Data communication
DISP. Display
GRAP. Graphics adapter
NET. Network adapter
RAM. Memory
STOR. Storage
ID1-ID2. Input device

### EXAMPLES

### Example 1 - Number of eye movements

Strain gauge sensors (SleepSense Limb Movement Sensors) from SleepSense were used as eye movement sensors. These where connected to a Digital Brain Electric Activity Mapping KT88 EEG apparatus as a recording and frequency analysis arrangement.

The subjects were instructed to keep their eyes closed, but to refrain from falling asleep.

The signal from the sensors where filtered by one of two different filters; a 2.5 Hz low pass filter with 24dB reduction.

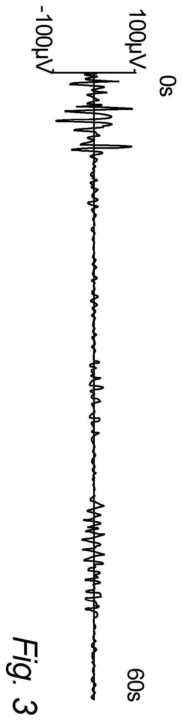

A signal was recorded over a period of 5 minutes. 1 minute of signal is illustrated in figure 3.

A large eye movement number was established as the number of times the signals exceeded 50 µV, or moved below -50 µV. This signifies the number of large eye movements.

As can be seen from figure 3, under these criteria, a number of eye movements are present during the beginning of the signal, whereas the rest of the signal which does not cross +/- 50 µV is not considered to contain eye movements under the above criteria.

### Table 1: Number of eye movements

**Table 1: Number of eye movements in 60 seconds intervals. The number of eye movements were identified as the number of times the eye movement signal (similar to that of figure 3) exceeded +/- 50 µV.**

| Patient ID | 1-60 s | 61-120 s | 121-180 s | 181-240 s | 241-300 s |
|---|---|---|---|---|---|
| 36 | 9 | 0 | 2 | 1 | 2 |
| 38 | 0 | 0 | 0 | 0 | 0 |
| 53 | 29 | 0 | 3 | 2 | 0 |
| 54 | 24 | 1 | 1 | 2 | 1 |
| 27 | 38 | 22 | 25 | 30 | 41 |
| 31 | 92 | 61 | 55 | 68 | 54 |

As can be seen from table 1, patients 27 and 31 displayed a significantly larger number of eye movements, especially in the latter four time intervals, i.e. after the first minute.

Patients 27 and 31 have been diagnosed with the psychiatric disorder ADHD, whereas patients 36, 38, 53, and 54 are healthy persons with no diagnosed psychiatric disorder. Patients 27 and 31 are unmedicated.

Thus, it can be seen that the number of eye movements shows significant difference between healthy persons and persons diagnosed with ADHD, which shows that the number of eye movements can be used to distinguish between persons having a normal dopamine/noradrenaline neurotransmitter balance and being healthy on one hand and persons with a dopamine/noradrenaline neurotransmitter imbalance having ADHD.

### Example 2 - Latency period

Using eye movement signals recorded as explained in example 1, the latency period was identified as follows.

The latency period is herein defined as the period of time of at least 10 seconds without eye movements.

This way of defining the latency time intends to account for situations, where the person is not actually trying to keep his/her eye still.

Since the 5 minutes (300 seconds) signals where used, latency periods of 300 seconds where maximum, corresponding to no movements of eyes.

Additionally, similar eye movement signals where recorded, with the difference that a 1.0 Hz low pass filter with 24dB reduction where used instead of that in example 1.

The results are shown in table 2.

### Table 2: Latency period

**Table 2: Latency period, being the first period of at least 10 seconds without eye movements. Shown for two different lowpass filters.**

| Patient ID | Latency period (seconds) | |
|---|---|---|
| | Lowpass 2.5 Hz | Lowpass 1.0 Hz |
| 36 | 141 | 293 |
| 38 | 300 | 300 |
| 53 | 30 | 300 |
| 54 | 53 | 300 |
| 27 | 16 | 113 |
| 31 | 9 | 14 |

As can be seen from table 2, the latency time shows a clear difference between persons with ADHD (27, 31), and healthy persons (36, 38, 53, 54). Patients 27 and 31 are unmedicated.

Thus, it can be seen that the latency time shows significant difference between healthy persons and persons diagnosed with ADHD, which shows that the latency time can be used to distinguish between persons having a normal dopamine/noradrenaline neurotransmitter balance and being healthy on one hand and persons with a dopamine/noradrenaline neurotransmitter imbalance having ADHD on the other hand.

### Example 3 - Frequency analysis of 5 minutes

Using eye movement signals recorded as explained in example 1, frequency analysis was performed to determine a frequency distribution. A Fast Fourier Transformation (FFT) based algorithm was used, with a 100 mHz resolution.

Results are shown in figures 4A-4F, for the persons 36, 38, 53, 54, 27, and 31, respectively.

As can be seen, a higher frequency content for ADHD-diagnosed persons 27, 31 were observed, compared to any of healthy control subjects 36, 38, 53, 54, 27.

Thus, it can be seen that the frequency content shows significant difference between healthy persons and persons diagnosed with ADHD, which shows that the frequency content can be used to distinguish between persons having a normal dopamine/noradrenaline neurotransmitter balance and being healthy on one hand and persons with a dopamine/noradrenaline neurotransmitter imbalance having ADHD on the other hand.

### Example 4 - Frequency analysis of 10 seconds

Using eye movement signals recorded as explained in example 1, frequency analysis was performed to determine a frequency distribution. However, only a 10 seconds period corresponding to the first 10 seconds of the latency time of example 2 was used. A Fast Fourier Transformation (FFT) based algorithm was used, with a 100 mHz resolution.

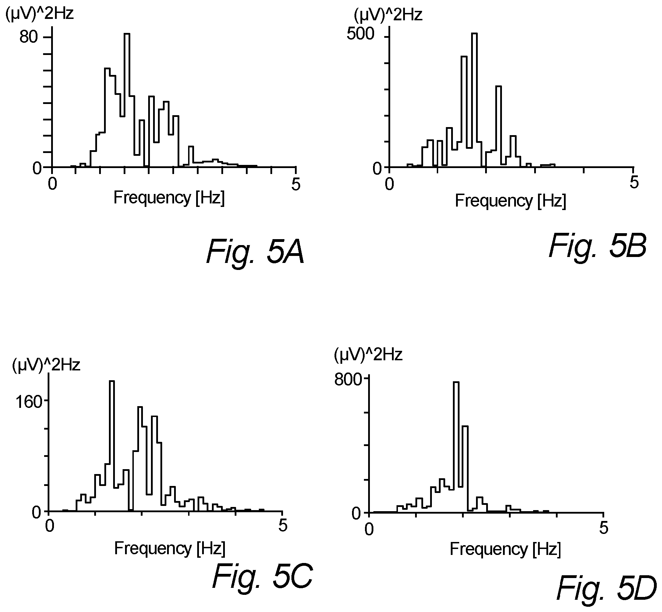

Results are shown in figure 5A-5D for person 53 (figure 5A), person 27 (figure 5B), person 54 (figure 5C), and person 31 (figure 5D).

As can be seen, a significantly higher frequency content is shown for persons with ADHD reaching much higher maximum values in the FFT diagram. Also, a frequency shift towards higher frequencies can be observed for persons with ADHD.

Thus, it can be seen that the frequency content shows significant difference between healthy persons and persons diagnosed with ADHD, which shows that the frequency content can be used to distinguish between persons having a normal dopamine/noradrenaline neurotransmitter balance and being healthy on one hand and persons with a dopamine/noradrenaline neurotransmitter imbalance having ADHD on the other hand.

Particularly, examples 3-4 illustrate that the selection of the time interval for the signal to frequency analyze may be important. Also, selecting a shorter signal time (e.g. 10 minutes instead of 5 minutes) surprisingly does not decrease accuracy, but perhaps even makes it easier to distinguish persons with ADHD from persons without ADHD.

### Example 5 - Measurements before/after treatment

Using eye movement signals recorded as explained in example 1, frequency analysis was performed to determine a frequency distribution. A Fast Fourier Transformation (FFT) based algorithm was used, with a 100 mHz resolution.

In example 5, signals were recorded on a patient with ADHD.

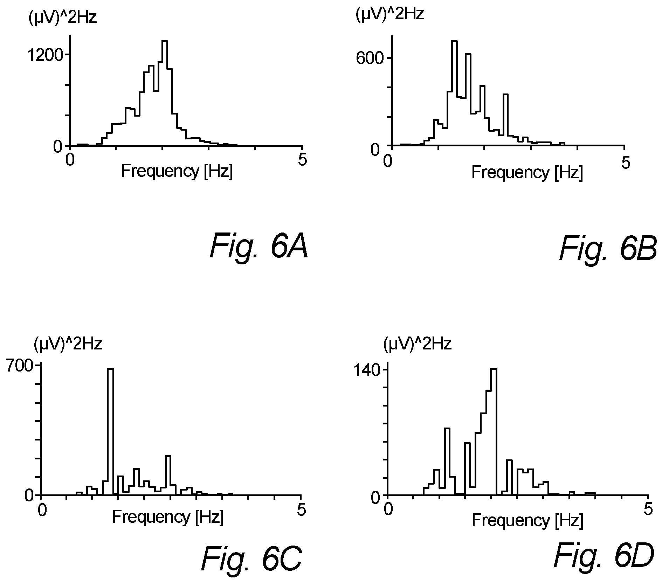

On figure 6A is the result when the patient was in an initial, unmedicated state. A second measurement was made after administering suitable ADHD-medication to the patient (one tablet of 10 mg of methylphenidate), this is shown in figure 6B.

Also, measurements were made as described in example 4, where figure 6C shows the result before medication, and figure 6D shows the result after the medication.

As can be seen when comparing figures 6A and 6B, the frequency content is lowered within the order of a reduction to 50%.

Similarly, figures 6C-6D show a significant reduction in frequency components, with a reduction of the maximum value within the order of a reduction to 20%.

Thus, it can be seen that the frequency content shows significant difference between medicated and unmedicated persons diagnosed with ADHD, which shows that the frequency content can be used to distinguish between persons having ADHD with an unmedicated dopamine/noradrenaline neurotransmitter imbalance on one hand and persons having ADHD with a medicated dopamine/noradrenaline neurotransmitter imbalance on the other hand.

Furthermore, the number of eye movements was analyzed as in example 1, before and after medication. The results are shown in table 3.

### Table 3 - Eye movements before/after medication

**Table 3: Number of eye movements in 60 seconds intervals. The number of eye movements were identified as the number of times the eye movement signal (similar to that of figure 3) exceeded +/- 50 µV.**

| | 1-60 s | 61-120 s | 121-180 s | 181-240 s | 241-300 s |
|---|---|---|---|---|---|
| Before medication | 54 | 15 | 23 | 16 | 14 |
| After medication | 60 | 0 | 2 | 1 | 2 |

As can be seen from table 3, the number of eye movements were significantly reduced, especially in the last four time intervals, i.e. after the first minute.

Thus, it can be seen that the number of eye movements shows significant difference between medicated and unmedicated persons diagnosed with ADHD, which shows that the number of eye movements can be used to distinguish between persons having ADHD with an unmedicated dopamine/noradrenaline neurotransmitter imbalance on one hand and persons having ADHD with a medicated dopamine/noradrenaline neurotransmitter imbalance on the other hand.

### Example 6

A further study was performed using a group consisting of 10 control subjects (i.e. healthy subjects), 7 subjects diagnosed with attention deficit hyperactivity disorder (ADHD), and 8 subjects diagnosed with attention deficit disorder (ADD).

Eye movement signals were recorded using the methods described in example 1.

### 6.1 Occurrence rate of large eye movements

First, a grouping of the subjects was performed according to the occurrence rate of large eye movements. In this context, a large eye movement is defined as giving an eye movement signal exceeding a certain predefined threshold. The groups were as follows:
A1: No pauses between temporally adjacent large eye movements of more than 5 seconds.
A2: At least one pause between temporally adjacent large eye movements above 5 seconds, but none above 20 seconds.
A3: At least one pause between temporally adjacent large eye movements above 20 seconds.

The grouping of the subjects into A1, A2, and A3 is shown in table 4.

### Table 4

**Table 4. Shows distribution of subjects between the three groups A1, A2, and A3, and the diagnostic groups (i.e. control, AHDH, ADD).**

| | Control | ADHD | ADD |
|---|---|---|---|
| A1 | 140 | 148 | |
| | 141 | 175 | |
| | 172 | 177 | |
| | | 179 | |
| | | 187 | |
| A2 | 137 | 143 | 154 |
| | 173 | | 157 |
| | | | 146 |
| A3 | 138 | 167 | 170 |
| | 139 | | 155 |
| | 142 | | 152 |
| | 150 | | 184 |
| | 151 | | 189 |

As can be seen from table 4, ADHD subjects tend to have shorter pauses between larger eye movements compared to control subjects, whereas ADD subjects tend to have longer pauses between large eye movements compared to control subjects.

### 6.2 Amplitudes of eye movement signal

The eye movement signals were analyzed to determine a representative amplitude. In this example, the sixth highest amplitude of the eye movement signal was used as representative amplitude. By not choosing the very largest, it is avoided that a single very high value among other much lower values disturbs the analysis.

Representative values are shown in table 5.

### Table 5

**Table 5. Representative amplitudes are shown for each subject. Subject id numbers are followed by their diagnosis, where K signifies a control subject.**

| | Representative amplitude [micro Volts] |
|---|---|
| 137 K | 160 |
| 138 K | 180 |
| 139 K | 80 |
| 140 K | 240 |
| 141 K | 220 |
| 142 K | 180 |
| 150 K | 120 |
| 151 K | 80 |
| 172 K | 100 |
| 173 K | 60 |
| 143 ADHD | 220 |
| 148 ADHD | 140 |
| 175 ADHD | 240 |
| 177 ADHD | 260 |
| 179 ADHD | 140 |
| 167 ADHD | 200 |
| 187 ADHD | 140 |
| 170 ADD | 40 |
| 146 ADD | 60 |
| 154 ADD | 80 |
| 157 ADD | 100 |
| 155 ADD | 40 |
| 152 ADD | 120 |
| 184 ADD | 100 |
| 189 ADD | 80 |

As can be seen from table 5, ADHD subjects have representative amplitudes above 140 µV. ADD subjects have representative amplitudes between 40 and 100 µV, and control subjects have representative amplitudes within 60 to 240 µV, i.e. within both the ADHD range and the ADD range.

It is believed that subjects with ADHD have excess noradrenalin relative to dopamine, and the representative amplitudes are believed to reflect this imbalance.

Subjects with ADD have a deficit of noradrenalin relative to dopamine, which result in the smaller representative amplitudes.

Since control subjects are randomly selected, it is assumed that when a subject is balanced, i.e. relaxed and not in a stressful state, the resulting representative amplitude would be found in the range between typical ADHD-values and ADD-values. However, when a person is in a stressful state, the instant noradrenalin-dopamine balance is shifted, and larger representative amplitudes result thereof.

On the other hand, when a person is tired, the noradrenalin levels decreases, leading the noradrenaline-dopamine balance to shift the other way, and the representative amplitudes to become smaller.

### 6.3 Further results

Further results obtained are given in tables 6-7.

### Table 6

**Table 6. "FFT Amp" signifies the maximum value of a fast Fourier transform (FFT) of the eye movement signal. "(FFT Amp)^2/Fq" signifies the squared value of FFT Amp, divided by "Fq", corresponding to the average frequency (shown as "Av Fq" in table 7). "Crossing" refers to the occurrence rate of zero crossings of the eye movement signal. Finally, "SUM" refers to the total sum of the values constituting the eye movement signal.**

| **Subject no.** | **Diagnosis** | **FFT Amp** | **(FFT Amp)^2/Fq** | **Crossing** | **SUM** |
|---|---|---|---|---|---|
| 137 | Control | 5.3 | 280 | 2692 | 391.487907 |
| 138 | Control | 4.4 | 190 | 2355 | 793.386133 |
| 139 | Control | 2.4 | 58 | 3049 | 392.388801 |
| 140 | Control | 11 | 1100 | 2041 | 1324.21210 |
| 141 | Control | 12 | 1500 | 1616 | 178.777147 |
| 142 | Control | 9.5 | 900 | 3916 | 879.771725 |
| 150 | Control | 4.5 | 250 | 3005 | -523.718925 |
| 151 | Control | 2.8 | 78 | 3298 | 588.683299 |
| 172 | Control | 5.5 | 310 | 2602 | 615.009381 |
| 173 | Control | 4.6 | 200 | 2230 | 520.215457 |
| 143 | ADHD | 15 | 2000 | 1945 | -11242.23941 |
| 148 | ADHD | 9.5 | 900 | 2152 | -105.202442 |
| 175 | ADHD | 23 | 5200 | 1348 | -14411.77996 |
| 177 | ADHD | 28 | 8000 | 1232 | 17357.999237 |
| 179 | ADHD | 9.5 | 900 | 1845 | 61.060502 |
| 167 | ADHD | 10.0 | 1000 | 2707 | -5970.515909 |
| 187 | ADHD | 9.5 | 910 | 1642 | 1853.136187 |
| 170 | ADD | 2.5 | 60 | 3483 | 927.819303 |
| 146 | ADD | 3.0 | 90 | 3046 | 958.950178 |
| 154 | ADD | 7.5 | 550 | 2115 | -2699.27486 |
| 157 | ADD | 6.0 | 400 | 2671 | -744.037232 |
| 155 | ADD | 3.9 | 150 | 3410 | 1159.949341 |
| 152 | ADD | 5.5 | 320 | 2177 | 1832.41566 |
| 184 | ADD | 5.1 | 260 | 2654 | 1645.230182 |
| 189 | ADD | 8.8 | 720 | 2277 | 382.779278 |

### Table 7

**Table 7. "Av amp" signifies the average amplitude. When this value is negative, it signifies predominant oscillation in negative direction. "Av Fq" signifies average frequency of the eye movement signal. "RMS" signifies the root mean square value of the amplitudes.**

| **Subject no.** | **Diagnosis** | **Av amp** | **Av Fq** | **RMS** |
|---|---|---|---|---|
| 137 | Control | +0.01305 | 4.486667 | 22.426845 |
| 138 | Control | +0.026446 | 3.925000 | 15.965592 |
| 139 | Control | +0.013080 | 5.081667 | 11.100932 |
| 140 | Control | +0.044140 | 3.401667 | 44.548648 |
| 141 | Control | +0.005959 | 2.693333 | 40.616152 |
| 142 | Control | +0.029326 | 6.52667 | 31.440807 |
| 150 | Control | -0.017457 | 5.008333 | 18.865063 |
| 151 | Control | +0.019623 | 5.49667 | 12.738009 |
| 172 | Control | +0.020500 | 4.336667 | 24.681106 |
| 173 | Control | +0.017341 | 3.716667 | 17.622235 |
| 143 | ADHD | -0.37474 | 3.241667 | 55.999352 |
| 148 | ADHD | -0.003507 | 3.58667 | 34.155251 |
| 175 | ADHD | -0.48393 | 2.246667 | 89.091864 |
| 177 | ADHD | +0.5786 | 2.053333 | 106.048559 |
| 179 | ADHD | +0.002030 | 3.07500 | 33.064496 |
| 167 | ADHD | -0.199017 | 4.511667 | 35.943681 |
| 187 | ADHD | +0.061771 | 2.736667 | 33.765549 |
| 170 | ADD | +0.030927 | 5.80555 | 8.968474 |
| 146 | ADD | +0.031965 | 5.07667 | 14.518534 |
| 154 | ADD | -0.089976 | 3.52500 | 25.326694 |
| 157 | ADD | -0.024801 | 4.451667 | 24.597789 |
| 155 | ADD | +0.038665 | 5.68333 | 13.508409 |
| 152 | ADD | +0.061081 | 3.62833 | 22.452989 |
| 184 | ADD | 0.054841 | 4.42333 | 21.075961 |
| 189 | ADD | 0.012759 | 3.79500 | 28.486843 |

### 6.4 Comparisons

### Using the data obtained in the above tables 4-7, the following comparisons are made

First, using the SUM values and the Crossing values from table 6, the subjects are mapped as shown in table 8.

### Table 8

**Table 8. Mapping of subjects as function of SUM and Crossing values.**

| | Crossing 0-2000 | Crossing 2000-3000 | Crossing 3000- |
|---|---|---|---|
| SUM < 100.000 | 175 ADHD | 167 ADHD | 150 K |
| | 143 ADHD | 154 ADD | |
| | 179 ADHD | 157 ADD | |
| | | 148 ADHD | |
| SUM 100.000-900.000 | 141 K | 137 K | 139 K |
| | | 173 K | 151 K |
| | | 172 K | 142 K |
| | | 138 K | |
| | | 189 ADD | |
| SUM **>** 900.000 | 187 ADHD | 140 K | 170 ADD |
| | 177 ADHD | 184 ADD | 146 ADD |
| | | 152 ADD | 155 ADD |

As can be seen from table 8, and ADD/ADHD subjects are within lower-moderate Crossing combined with low SUM, or within the high SUM range; except for the following:
140 and 150 can be seen as fake positives, whereas 189 is not detected. It is noted that by looking at the frequency content around 10Hz of the FFT of the eye movement signals, it was noted that 140 displayed much lower values than average ADHD subjects. Also, 189 was further diagnosed with severe eating disorder, anxiety condition and obsessive compulsion disorder (OCD).

Secondly, a comparison between average amplitude and occurrence rate of zero crossings was made, where the results are shown in table 9.

### Table 9

**Table 9. Mapping of subjects as function of average amplitude and Crossing values.**

| Average amplitude | Crossing 0-2000 | Crossing 2000-3000 | Crossing above 3000 |
|---|---|---|---|
| 10-0.03 | 177 ADHD | 140 K | 170 ADD |
| | 187 ADHD | 152 ADD | 146 ADD |
| | | 184 ADD | 155 ADD |
| 0.005-0.03 | 141 K | 137 K | 139 K |
| | | 138 K | 142 K |
| | | 172 K | 151 K |
| | | 173 K | |
| | | 189 ADD | |
| -10- 0.005 | 143 ADHD | 148 ADHD | 150 K |
| | 175 ADHD | 167 ADHD | |
| | 179 ADHD | 154 ADD | |
| | | 157 ADD | |

As can be seen from table 9, similar results as for the mapping of table 8 may be obtained, most cases of ADHD/ADD are in the range of above 0.03 or below 0.005 for the average amplitude. Again, subjects 140, 150, and 189 are incorrectly identified, as for table 8.

As it can be seen from table 9, typical test subjects would give rise to lowest magnitude of average amplitudes.

Then, a comparison between average amplitude and maximum FFT amplitude was made, where the results are shown in table 10.

### Table 10

**Table 10. Mapping of subjects as function of average amplitude and maximum FFT amplitudes. Here, FFT amp refers to the maximum value of a fast Fourier transform (FFT) of the eye movement signal, as in table 6.**

| Average amplitude | FFT amp 0-6 | FFT amp 6-12 | FFT amp 12-30 |
|---|---|---|---|
| 10-0.03 | 170 ADD | 140 K | 177ADHD |
| | 146 ADD | 187ADD | |
| | 155 ADD | | |
| | 152 ADD | | |
| | 184 ADD | | |
| 0.005-0.03 | 137 K | 141 K | |
| | 138 K | 142 K | |
| | 139 K | | |
| | 151 K | | |
| | 172 K | | |
| | 173 K | | |
| -10- 0.005 | 150 K | 148 ADHD | 143 ADHD |
| | | 179 ADHD | 175 ADHD |
| | | 167 ADHD | |
| | | 154 ADD | |
| | | 157 ADD | |

As can be seen from table 10, most subjects having ADHD/ADD can be correctly identified.

Then, a comparison between above described groups A1, A2, and A3 and the total sum of the values constituting the eye movement signal (SUM) was made, where the results are shown in table 11.

### Table 11

**Table 11. Mapping of subjects as function of group A1, A2, and A3 and total sum of the values constituting the eye movement signal (SUM).**

| Group | SUM <100.000 | 100.000-900.000 | SUM>900.000 |
|---|---|---|---|
| A1 | 175 ADHD | 141 K | 187 ADHD |
| | 179 ADHD | 172 K | 177 ADHD |
| | 148 ADHD | | 140 K |
| A2 | 143 ADHD | 137 K | |
| | 154 ADD | 173 K | |
| | 157 ADD | | |
| A3 | 150 K | 138 K | 184 ADD |
| | 167 ADHD | 151 K | 152 ADD |
| | | 142 K | 155 ADD |
| | | 139 K | 170 ADD |
| | | 189 ADD | 146 ADD |

As can be seen from table 11, most subjects having ADHD/ADD can be correctly identified.

Then, a comparison between above described groups A1, A2, and A3 and the average amplitude of the eye movement signal was made, where the results are shown in table 12.

### Table 12

**Table 12. Mapping of subjects as function of group A1, A2, and A3 and the average amplitude of the eye movement signal was made.**

| | Av-amp 10-0.03 | Av-amp 0.005-0.03 | Av-amp -10-0.005 |
|---|---|---|---|
| A1 | 177 ADHD | 141 K | 175 ADHD |
| | 187 ADHD | 172 K | 179 ADHD |
| | 140 K | | 148 ADHD |
| A2 | | 137 K | 143 ADHD |
| | | 173 K | 154 ADD |
| | | | 157 ADD |
| A3 | 152 ADD | 138 K | 167 ADD |
| | 184 ADD | 151 K | 150 K |
| | 170 ADD | 142 K | |
| | 146 ADD | 139 K | |
| | 155 ADD | 189 ADD | |

As can be seen from table 12, most subjects having ADHD/ADD can be correctly identified.

As seen from the above, ADHD/ADD subjects may be identified in a number of different ways, with a relatively high degree of certainty, comparing to other available and typically subjective diagnostic criteria.

However, ADD subjects and ADHD subjects are typically difficult to distinguish using the methods described above in example 6.

### 6.5 Use of frequency analysis

Fast Fourier transforms (FFT) were made for eye movement signals recorded from subjects. It was observed that the FFTs of ADHD had considerably higher frequency contents within certain ranges, compared to ADD subjects.

It was also noted that when squaring the FFT values and dividing by the respective frequency, and even more significant deviation between ADHD subjects and ADD subjects was observed.

### 6.6 Treatment of ADHD and ADD

Subjects with ADHD have a too high level of noradrenalin relative to dopamine. Thus, an input of dopamine could theoretically correct this imbalance.

### Table 13

**Table 13. Shows data of ADHD subjects before (Data A) and after (Data B) treatment with methylphenidate. FFT Amplitude refers to the maximum value of a fast Fourier transform (FFT) of the eye movement signal. Av amp" signifies the average amplitude. When this value is negative, it signifies predominant oscillation in negative direction. "Av Fq" signifies average frequency of the eye movement signal.**

| | DATA A | | | DATA B | | |
|---|---|---|---|---|---|---|
| | FFT Amplitude | Av Amp | Av Fq | FFT Amplitude | Av Amp | Av Fq |
| 143/144 | 15 | -0.37474 | 3.241667 | 7 | 0.0011375 | 4.858333 |
| 177/178 | 28 | 0.578600 | 2.05333 | 26 | 0.505568 | 2.23000 |
| 179/180 | 9.5 | 0.002030 | 3.07500 | 4.1 | 0.049990 | 3.69500 |
| 187/188 | 9.5 | 0.061771 | 2.73667 | 12 | 0.092632 | 2.55333 |
| 189/190 | 8.5 | 0.012759 | 3.79500 | 5.0 | 0.007571 | 3.946667 |
| 167/168 | 10 | -0.199017 | 4.511667 | 9.6 | 0.044788 | 4.646667 |
| 192/193 | 22 | 0.046206 | 2.425000 | 8.0 | 0.000667 | 4.838333 |

Data from 7 subjects with ADHD examined before treatment. Hereafter methylphenidate was administered, and after a subjective assessment of an effect thereof, the measurement was repeated. The sensors were in place during the entire test.

It is observed that in 6 cases a fall in the amplitude occurs. At the same time, an increase in the average frequency is observed.

One case of an increase in amplitude and decrease in frequency was observed.

Subjects with ADD have a too high level of dopamine relative to noradrenalin. By adding dopamine, a worsening of the condition should therefore be expected.

### Table 14

**Table 14. Shows data of ADD subjects before (Data A) and after (Data B) treatment with methylphenidate. FFT Amplitude refers to the maximum value of a fast Fourier transform (FFT) of the eye movement signal. Av amp" signifies the average amplitude. When this value is negative, it signifies predominant oscillation in negative direction. "Av Fq" signifies average frequency of the eye movement signal.**

| | DATA A | | | DATA B | | |
|---|---|---|---|---|---|---|
| | FFT Amplitude | Av Amp | Av Fq | FFT Amplitude | Av Amp | Av Fq |
| 146/147 | 3 | 0.031965 | 5.07667 | 6.1 | 0.032752 | 4.08333 |
| 170/171 | 2.5 | 0.030927 | 5.80555 | 2.6 | 0.025912 | 5.52333 |
| 184/185 | 5.0 | 0.054841 | 4.42333 | 8.0 | 0.054724 | 3.64000 |
| 152/153 | 5.5 | 0.061080 | 3.62833 | 4.2 | 0.048525 | 4.61833 |

Table 14 shows 4 subjects treated with methylphenidate, which increases the dopamine levels in the brain. In 3 out of 4 cases, an increase in the amplitude and a decrease in the average frequency was observed.

In one case, a decrease in amplitude and an increase in frequency was observed.

In most cases in table 13-14, the administration of methylphenidate gave the expected results.

It is noted that the subject in table 13 reacting opposite to other subjects may represent a case an erroneous diagnosis, i.e. a subject which should have been diagnosed with ADD. Similarly, the one case reacting opposite to the other subjects in table 14 may represent a case an erroneous diagnosis, i.e. a subject which should have been diagnosed with ADHD.

### 6.7 Imbalance detection

It is hypothesized that the ratio between the average amplitude and the average frequency is a measure for the ratio between the dopamine level and the noradrenalin level of the brain. Thus, a deviation in the ratio between the average amplitude and the average frequency would signify an imbalance in the dopamine-noradrenaline levels.

### Table 15

**Table 15. Values of ratio between average amplitude and average frequency.**

| **Subject no.** | **Diagnose** | **Av - amp** | **Av-Fq** | **Av - amp / Av - F** |
|---|---|---|---|---|
| 137 | Kontrol | +0.01305 | 4.486667 | 345 |
| 138 | Kontrol | +0.026446 | 3.925000 | 150 |
| 139 | Kontrol | +0.013080 | 5.081667 | 390 |
| 140 | Kontrol | +0.044140 | 3.401667 | 77 |
| 141 | Kontrol | +0.005959 | 2.693333 | 448 |
| 142 | Kontrol | +0.029326 | 6.52667 | 225 |
| 150 | Kontrol | -0.017457 | 5.008333 | -294 |
| 151 | Kontrol | +0.019623 | 5.49667 | 274 |
| 172 | Kontrol | +0.020500 | 4.336667 | 173 |
| 173 | Kontrol | +0.017341 | 3.716667 | 218 |
| 143 | ADHD | -0.37474 | 3.241667 | -8 |
| 148 | ADHD | -0.003507 | 3.58667 | -896 |
| 175 | ADHD | -0.48393 | 2.246667 | -4 |
| 177 | ADHD | +0.5786 | 2.053333 | 3 |
| 179 | ADHD | +0.002030 | 3.07500 | 1537 |
| 167 | ADHD | -0.199017 | 4.511667 | -22 |
| 187 | ADHD | +0.061771 | 2.736667 | 44 |
| 170 | ADD | +0.030927 | 5.80555 | 187 |
| 146 | ADD | +0.031965 | 5.07667 | 158 |
| 154 | ADD | -0.089976 | 3.52500 | -39 |
| 157 | ADD | -0.024801 | 4.451667 | -178 |
| 155 | ADD | +0.038665 | 5.68333 | 145 |
| 152 | ADD | +0.061081 | 3.62833 | 59 |
| 184 | ADD | 0.054841 | 4.42333 | 80 |
| 189 | ADD | 0.012759 | 3.79500 | 316 |

These values may also be seen in the mapping shown in table 16.

### Table 16

**Table 16. Mapping of subjects as function of average amplitude (Av amp) and the average frequency (Av Fq). Values of average amplitude shown in parenthesis**

| | Av-Fq: 0 - 3 | Av-Fq: 3 - 4 | Av-Fq: above 4 |
|---|---|---|---|
| Av-amp lower than 0.005 | 175 ADHD (-0.48) | 143 ADHD (-3.7) | 150K (- 0.017) |
| | 179 ADHD (0.002) | 148 ADHD (-0.003) | 167 ADHD (-0.199) |
| | | 154 ADD (-0.089 | |
| | | | 157 ADD (-0.024) |
| Av-amp 0.005 - 0.03 | 141K (0.0059) | 138K (0.026) | 137K (0.013) |
| | | 173K (0.017) | 139K (0.013) |
| | | 189 ADD | 142K (0.029) |
| | | | 151K (0.019) |
| | | | 172K (0.017) |
| Av-amp at least 0.03 | 177 ADHD (0.58) | 140K (0.044) | 170 ADD (0.030) |
| | 187 ADHD (ADD) (0.061) | 152 ADD (ADHD) (0.061) | 146 ADD (0.031) |
| | | | 155 ADD (0.038) |
| | | | 184 ADD (0.054) |

This table shows similar results as the mapping in example 6.4.

### Example 7

### 7.1 Evaluation of sensor response

Sensor response was evaluated.

Two types of movements of the sensor was evaluated. First, movements with smaller deflection was compared with movements with larger deflections. Secondly, slower movements were compared with faster movements.

Not surprisingly, larger movements generated a larger signal (e.g. in terms of maximum amplitude) than smaller deflection movements. Similarly, faster movements generated a larger signal (e.g. in terms of maximum amplitude) than slower movements

### 7.2 Signal recording and processing

A number of sample signals were recorded. Each sample was recorded over a period of 360 seconds. Only the middle 240 seconds are used, as the first 60 seconds and the last 60 second of the signal is discarded to avoid introducing artifacts related to the startup and termination of the test and the subject getting used to the eye sensors etc.

The signals were subjected to frequency analysis by FFT with 100 mHz solution.

### 7.3 Signal recording and processing

Maximum amplitude of the frequency distribution (i.e. max FFT amplitude) is identified.

20 subjects with an AHDH or ADD diagnosis were evaluated with respect to effect of startup of methylphenidate treatment. A sample was recorded for each subject in accordance with example 7.2, and a maximum FFT amplitude was outputted from the frequency analysis arrangement (maximum FFT amplitude before, MAB). Thereafter, a startup dose of methylphenidate was prescribed for each subject. After ingestion, a further sample was recorded for each subject, and a maximum FFT amplitude was outputted from the frequency analysis arrangement (maximum FFT amplitude after, MAA). The results are shown in table 17.

### Table 17

**Table 17. SI/D: Subject identifier/diagnosis. MAB: Maximum FFT amplitude before. MAA: Maximum FFT amplitude after. RC: relative change in FFT amplitude. Eff. M.: Evaluation of effect of methylphenidate. C/SE: Comorbidity / side effects**

| **SI/D** | **MAB** | **MAA** | **RC** | **Eff. M.** | **C/SE** | **Final treatment** |
|---|---|---|---|---|---|---|
| 202 ADHD | 34.4 | 45.9 | 33 | No | - | Atomoxetine |
| 177 ADHD | 26.7 | 24.8 | -7 | No | Depression | No effect of ADHD treatment |
| 192 ADHD | 21.4 | 7.3 | -66 | Good | - | Methylphenidate |
| 255 ADD | 22.9 | 16.0 | -30 | Good | - | Methylphenidate |
| 248 ADHD | 15.9 | 18.2 | 14 | No | - | No effect of ADHD treatment |
| 250 ADD | 15.8 | 12.0 | -24 | Good | Depression | Methylphenidate |
| 197 ADD | 14.2 | 6.2 | -56 | Good | Depression | Methylphenidate |
| 212 ADD | 13.1 | 16.7 | 27 | No | - | No effect of ADHD treatment |
| 143 ADHD | 14.3 | 6.7 | -53 | Good | Psychosis | No treatment |
| 258 ADD | 11.2 | 11.0 | -2 | No | Depression | Atomoxetine |
| 187 ADHD | 9.0 | 10.7 | 19 | Good | Addiction | No treatment |
| | | | | | | |
| 148 ADHD | 8.7 | 3.5 | -60 | Good | - | Methylphenidate |
| 179 ADHD | 8.9 | 3.1 | -65 | Good | - | Lisdexamphetamine |
| 210 ADHD | 7.6 | 8.7 | 14 | No | Anxiety | No effect of ADHD treatment |
| 155 ADD | 3.6 | 4.2 | 17 | No | Depression | No effect of ADHD treatment |
| 204 ADHD | 6.2 | 2.1 | -66 | Good | - | Lisdexamphetamine |
| 189 ADD | 9.0 | 5.5 | -39 | Good | OCD, Anxiety | Lisdexamphetamine |
| 184 ADD | 4.8 | 7.9 | 65 | Good | - | No effect of ADHD treatment |
| 152 ADD | 4.58 | 2.0 | -56 | Good | - | Methylphenidate |
| 146 ADD | 2.8 | 5.6 | 100 | No | Depression | Atomoxetine |

As can be seen from table 17, some subjects showed a significant reduction in maximum FFT amplitude, whereas others showed no significant change or even increase. A reduction of the maximum FFT amplitude indicates a reduced eye activity. Comparing the evaluated effect of the methylphenidate with the change in eye activity (RC), it is seen that except for subjects 187 and 184, a complete correspondence between a good effect and a reduction in eye activity (RC) is observed.

Subjects 177 and 258 both showed a small decrease in eye activity (RC) below 10% and had not positive effect of the treatment.

Depending on the comorbidity / side effects (C/SE), it could be decided whether to continue or terminate treatment or to try an alternative drug, such as atomoxetine or lisdexamphetamine.

### 7.4 Evaluation of correlation between peak FFT amplitude and diagnosis.

20 subjects diagnosed with AHDH or ADD or begin control subjects without diagnosis were investigated. A sample was recorded for each subject in accordance with example 7.2, and a peak FFT amplitude was outputted from the frequency analysis arrangement.

Also, the average frequency (Av. freq.), the RMS, and the number of zero crossings were determined and listed in table 18.

### Table 18

**Table 18. Value for 20 subjects. Ranked by peak FFT amplitude. Diagnosis is indicated after each subject identifier, where K indicates a control subject. SI/D: Subject identifier/diagnosis.**

| **SI/D** | **Peak FFT amplitude** | **Av. freq.** | **RMS** | **No. of zero crossing** |
|---|---|---|---|---|
| 202 ADHD | 34.44 | 1.99 | 120.32 | 935 |
| 177 ADHD | 26.7 | 2.13 | 99.25 | 1023 |
| 255 ADD | 22.93 | 2.32 | 79.39 | 1416 |
| 192 ADHD | 21.48 | 2.48 | 81.31 | 1191 |
| 248 ADD | 16.98 | 3.47 | 74.26 | 2471 |
| 250 ADD | 15.81 | 3.25 | 61.12 | 1568 |
| 143 ADHD | 14.35 | 3.8 | 52.2 | 1927 |
| 197 ADHD | 14.26 | 2.88 | 52.67 | 1387 |
| 212 ADHD | 13.16 | 2.46 | 54.75 | 1184 |
| 258 ADD | 11.22 | 3.35 | 38.55 | 1614 |
| 141 K | 10.34 | 3.05 | 32.94 | 1466 |
| 189 ADD | 9.06 | 3.85 | 28.57 | 1860 |
| 187 ADHD | 9.05 | 2.96 | 30.07 | 1421 |
| 179 ADHD | 8.97 | 3.47 | 29.43 | 1669 |
| 148 ADHD | 8.73 | 3.94 | 29.66 | 1894 |
| 210 ADHD | 7.67 | 3.17 | 31.36 | 1523 |
| 204 ADD | 6.29 | 4.43 | 22.61 | 2129 |
| 246 K | 5.69 | 3.43 | 20.55 | 1651 |
| 172 K | 5.66 | 4.01 | 22.96 | 1931 |
| 142 K | 5.32 | 7.48 | 17.53 | 3593 |
| 184 ADD | 4.86 | 4.59 | 19.35 | 2207 |
| 137 K | 4.8 | 5.01 | 19.93 | 2407 |
| 152 ADD | 4.58 | 4.22 | 16.04 | 2026 |
| 173 K | 4.52 | 4.12 | 16.59 | 1982 |
| 150 K | 4.27 | 5.92 | 15.76 | 2843 |
| 155 ADD | 3.6 | 5.79 | 11.92 | 2879 |
| 138 K | 2.95 | 6.23 | 9.95 | 2991 |
| 151 K | 2.48 | 6.15 | 11.23 | 2956 |
| 146 ADD | 2.32 | 5.14 | 13.93 | 1519 |
| 139 K | 2.21 | 5.18 | 10.22 | 2489 |
| | | | | |
| Average K | 4.39 | 5.05 | 17.76 | 2430 |
| Average ADHD | 15.87 | 2.82 | 58.10 | 1415 |
| Average ADD | 9.76 | 4.04 | 36.57 | 1968 |

The entries in table 18 are ranked by peak FFT amplitude. This shows a very clear tendency that all ADHD display a peak FFT amplitude above a threshold value of about 6-7.

Also, all but one of the control subjects displayed a peak FFT amplitude below the threshold value of about 6-7.

Finally, no clear threshold can be seen in view of the ADD subjects, even though the average peak FFT amplitude for ADD subjects of 9.76 were significantly higher than for control subjects with average of 4.39. However, ADHD subjects showed an even higher average peak FFT amplitude of 15.87, consistent with the clearer delimitation relative to control subjects.

Further, the average frequency is lowest for the ADHD subjects consistent with a relatively high contribution from eye movements in the 1-5Hz range compared to a background noise from eye lid muscle behavior e.g. in the 10-20Hz range.

Finally, ADHD subjects showed the highest average RMS-value and the lowest average number of zero crossings.

### Table 19

**Table 19. Value for three subjects with severe depression. Ranked by peak FFT amplitude. SI/D: Subject identifier/diagnosis.**

| **SI/D** | **Peak FFT amplitude** | **Av. freq.** | **RMS** | **No. of zero crossing** |
|---|---|---|---|---|
| 208 | 9.5 | 4.08 | 38.66 | 1470 |
| 209 | 13.5 | 2.355 | 47.15 | 847 |
| 227 | 27 | 2.79 | 113.83 | 1007 |

As can be seen from table 19, the three subjects all scored high on the peak FFT amplitude (samples measured in accordance with example 7.2), above threshold of e.g. 6-7, indicating that the peak FFT amplitude cannot discriminate between subjects with ADHD and severe depression.

In other words, the present disclosure provides a tool to advance towards a diagnosis (i.e. it is diagnostically relevant) but cannot be used by itself to provide a diagnosis, as several different diseases may be the source of the deviation from normal parameters.

It is particularly believed that ADHD, ADD, stress disorder, depression, anxiety, obsessive-compulsive disorder (OCD), post-traumatic stress disorder (PTSD), and schizophrenia will score high values e.g. at peak FFT amplitude. To distinguish these disorders from each other a number of different tools may be applied. This may include conventional psychiatric diagnostic tools, but also physiological measurements such as e.g. pulse to distinguish between stress (typically faster pulse) and ADHD (typically normal level pulse).

It is believed that the above diagnoses, particularly AHDH and ADD, are results of a dopamine-noradrenaline imbalance, and that the frequency density indication signal indicating a frequency density in e.g. a 1-5Hz range is an indication of a high eye movement activity, which again indicates a dopamine-noradrenaline imbalance.

### 7.5 Evaluation of correlation between peak FFT amplitude and diagnosis

To evaluate the relevance of the frequency range used, FFT distributions for three control subjects were compared to with FFT distributions for three subjects with depressions in the frequency range of 10-20Hz. Samples were recorded in accordance with example 7.2

Control subjects scored peak FFT amplitude in the 10-20Hz range of 0.17 to 0.26, whereas subjects with depression scored at least 0.44, i.e. approximately twice as high as control subjects.

It is believed that this frequency range (10-20Hz) correlates more with muscle activity in the eye lid rather than eye movements, whereas eye movements predominantly give rise to frequency components in the 1-5Hz range.

### 7.6 Evaluation of reproducibility

To evaluate the degree of reproducibility, an FFT distribution was recorded several times in accordance with example 7.2 for the same subject at different points of time over a total period of several days. Parameters were calculated and shown in table 20.

### Table 20

**Table 20. Comparable values obtained for the same subject over time. SI/D: Subject identifier/diagnosis.**

| **SI/D** | **Peak FFT amplitude** | **Av. freq.** | **RMS** | **No. of zero crossing** |
|---|---|---|---|---|
| 138 K, 1 | 1.6 | 6.50 | 6.05 | 2341 |
| 138 K, 2 | 1.6 | 7.4 | 6.91 | 2666 |
| 138 K, 3 | 2.0 | 6.85 | 8.72 | 2469 |
| 138 K, 4 | 7.5 | 5.51 | 23.01 | 1984 |
| 138 K, 5 | 3.7 | 7.11 | 15.35 | 2562 |
| 138 K, 6 | 2.8 | 5.41 | 11.01 | 1957 |
| 138 K, 7 | 2.8 | 5.85 | 9.55 | 2108 |
| 138 K, 8 | 6.5 | 2.98 | 24.95 | 1076 |
| 138 K, 9 | 1.5 | 6.07 | 6.20 | 2187 |
| 138 K, 10 | 2.6 | 7.84 | 6.22 | 2823 |
| 138 K, 11 | 1.4 | 8.04 | 4.72 | 2896 |
| 138 K, 12 | 2.1 | 7.2 | 6.73 | 2594 |
| 138 K, 13 | 2.4 | 7.79 | 9.19 | 2804 |
| 138 K, 14 | 2.4 | 5.73 | 8.58 | 2066 |

As can be seen from table 20, relatively consistent measurements where obtained, with some deviation. This indicates that average values taken at different points of time appear to be more accurate than individual values. E.g. while two peak FFT amplitudes exceeds 6, the average value of 2.9 is well below this threshold.

## Claims

1. A neurotransmitter imbalance detection system (SYS),
said system (SYS) comprising
at least one eye movement sensor (SEN) for sensing movement of a closed eye (EYE),
a frequency analysis arrangement (FAA), and
wherein said eye movement sensor (SEN) is configured to output at least one eye movement signal (EMS) representing movement of the closed eye (EYE) and to communicate said at least one eye movement signal (EMS) to said frequency analysis arrangement (FAA),
wherein said frequency analysis arrangement (FAA) is configured to
receive said at least one eye movement signal (EMS) and
process said at least one eye movement signal (EMS) by frequency analysis to determine a frequency distribution,
output a frequency density indication signal (FDIS) correlating with a frequency density within a predefined frequency range, and to
determine if said frequency density indication signal (FDIS) exceeds a predetermined threshold,
identifying an abnormal condition when the threshold is exceeded,
wherein the neurotransmitter imbalance is associated with Attention Deficit Hyperactivity Disorder (ADHD),
wherein the predefined frequency range comprises at least the range from 1 to 3 Hz, and
wherein the predetermined threshold is at least 10% of the total frequency content.

2. The neurotransmitter imbalance detection system (SYS) according to claim 1, wherein the frequency analysis arrangement (FAA) is further configured to output a frequency content signal (FCS) representing a frequency content within a predefined frequency range.

3. The neurotransmitter imbalance detection system (SYS) according to claim 1 or 2, wherein the system (SYS) further comprises an oscillation analysis arrangement, the oscillation analysis arrangement, the oscillation analysis arrangement being configure to
receive said at least one eye movement signal (EMS), and
process said at least one eye movement signal (EMS).

4. The neurotransmitter imbalance detection system (SYS) according to claim 3, wherein the oscillation analysis arrangement is configured to
process said at least one eye movement signal (EMS) to determine the occurrence rate of oscillations exceeding a predetermined threshold.

5. The neurotransmitter imbalance detection system (SYS) according to claim 3 or 4, wherein the oscillation analysis arrangement is configured to
process said at least one eye movement signal (EMS) by to determine a representative amplitude of eye movements associated with an oscillation of the eye movement signal exceeding a predetermined threshold.

6. The neurotransmitter imbalance detection system (SYS) according to any of claims 3-5, wherein the oscillation analysis arrangement is configured to
process said at least one eye movement signal (EMS) by to determine the occurrence rate of zero crossings of the eye movement signal (EMS).

7. The neurotransmitter imbalance detection system (SYS) according to any of claims 3-6, wherein the oscillation analysis arrangement is configured to
process said at least one eye movement signal (EMS) by to determine the total sum of the eye movement signal (EMS).

8. The neurotransmitter imbalance detection system (SYS) according to any of claims 1-7, wherein the system is further configured to determine the maximum value of the frequency distribution.

9. The neurotransmitter imbalance detection system (SYS) according to any of claims 1-8, wherein said frequency analysis arrangement (FAA) is further configured to compare said frequency content signal with a normal range representation and an abnormal range representation.

10. The neurotransmitter imbalance detection system (SYS) according to any of claims 1-9, wherein said frequency analysis arrangement (FAA) is further configured to determine if relative content in said predefined frequency range exceeds a predetermined threshold.

11. The neurotransmitter imbalance detection system (SYS) according to any of claims 1-10, wherein the predefined frequency range comprises at least the range from 1 Hz to 5 Hz.

12. The neurotransmitter imbalance detection system (SYS) according to any of claims 1-11, wherein the system (SYS) further comprising an analysis unit configured to identify the number of eye movement events within a predefined time range.

13. The neurotransmitter imbalance detection system (SYS) according to any of claims 1-12, wherein the at least one eye movement sensor (SEN) comprises a strain gauge sensor.

14. The neurotransmitter imbalance detection system (SYS) according to any of claims 1-13, wherein the system (SYS) is configured to output an evaluation value indicating a degree of positive response to a medication, the evaluation value being based on at least
- a first eye movement signal (EMS) recorded prior to intake of the medication and
- a second eye movement signal (EMS) recorded after intake of the medication.

## Patentansprüche

1. Neurotransmitterungleichgewichterkennungssystem (SYS), das System (SYS) umfassend
mindestens einen Augenbewegungssensor (SEN) zum Erfassen einer Bewegung eines geschlossenen Auges (EYE),
eine Frequenzanalyseanordnung (FAA), und
wobei der Augenbewegungssensor (SEN) konfiguriert ist, um mindestens ein Augenbewegungssignal (EMS) auszugeben, das die Bewegung des geschlossenen Auges (EYE) darstellt, und um das mindestens eine Augenbewegungssignal (EMS) an die Frequenzanalyseanordnung (FAA) zu kommunizieren,
wobei die Frequenzanalyseanordnung (FAA) konfiguriert ist zum
Empfangen des mindestens einen Augenbewegungssignals (EMS) und
Verarbeiten des mindestens einen Augenbewegungssignals (EMS) durch Frequenzanalyse, um eine Frequenzverteilung zu bestimmen,
Ausgeben eines Frequenzdichteangabesignals (FDIS), das mit einer Frequenzdichte innerhalb eines vordefinierten Frequenzbereichs korreliert, und zum
Bestimmen, ob das Frequenzdichteangabesignal (FDIS) einen vorbestimmten Schwellenwert überschreitet,
Identifizieren eines anormalen Zustands, wenn der Schwellenwert überschritten wird,
wobei das Neurotransmitterungleichgewicht mit Aufmerksamkeitsdefizit-/Hyperaktivitätsstörung (ADHS) assoziiert ist,
wobei der vordefinierte Frequenzbereich mindestens den Bereich von 1 bis 3 Hz umfasst, und
wobei der vorbestimmte Schwellenwert mindestens 10 % des gesamten Frequenzgehalts beträgt.

2. Neurotransmitterungleichgewichterkennungssystem (SYS) nach Anspruch 1, wobei die Frequenzanalyseanordnung (FAA) ferner konfiguriert ist, um ein Frequenzgehaltsignal (FCS) auszugeben, das einen Frequenzgehalt innerhalb eines vordefinierten Frequenzbereichs darstellt.

3. Neurotransmitterungleichgewichterkennungssystem (SYS) nach Anspruch 1 oder 2, wobei das System (SYS) ferner eine Oszillationsanalyseanordnung umfasst, die Oszillationsanalyseanordnung, wobei die Oszillationsanalyseanordnung konfiguriert ist zum
Empfangen des mindestens einen Augenbewegungssignals (EMS), und
Verarbeiten des mindestens einen Augenbewegungssignals (EMS).

4. Neurotransmitterungleichgewichterkennungssystem (SYS) nach Anspruch 3, wobei die Oszillationsanalyseanordnung konfiguriert ist zum
Verarbeiten des mindestens einen Augenbewegungssignals (EMS), um die Auftrittsrate von Oszillationen zu bestimmen, die einen vorbestimmten Schwellenwert überschreiten.

5. Neurotransmitterungleichgewichterkennungssystem (SYS) nach Anspruch 3 oder 4, wobei die Oszillationsanalyseanordnung konfiguriert ist zum
Verarbeiten des mindestens einen Augenbewegungssignals (EMS), um eine darstellende Amplitude von Augenbewegungen zu bestimmen, die mit einer Oszillation des Augenbewegungssignals assoziiert sind, die einen vorbestimmten Schwellenwert überschreitet.

6. Neurotransmitterungleichgewichterkennungssystem (SYS) nach einem der Ansprüche 3 bis 5, wobei die Oszillationsanalyseanordnung konfiguriert ist zum
Verarbeiten des mindestens einen Augenbewegungssignals (EMS), um die Auftrittsrate von Nulldurchgängen des Augenbewegungssignals (EMS) zu bestimmen.

7. Neurotransmitterungleichgewichterkennungssystem (SYS) nach einem der Ansprüche 3 bis 6, wobei die Oszillationsanalyseanordnung konfiguriert ist zum
Verarbeiten des mindestens einen Augenbewegungssignal (EMS), um die Gesamtsumme des Augenbewegungssignals (EMS) zu bestimmen.

8. Neurotransmitterungleichgewichterkennungssystem (SYS) nach einem der Ansprüche 1 bis 7, wobei das System ferner konfiguriert ist, um den maximalen Wert der Frequenzverteilung zu bestimmen.

9. Neurotransmitterungleichgewichterkennungssystem (SYS) nach einem der Ansprüche 1 bis 8, wobei die Frequenzanalyseanordnung (FAA) ferner konfiguriert ist, um das Frequenzgehaltsignal mit einer Normalbereichsdarstellung und einer Abnormalbereichsdarstellung zu vergleichen.

10. Neurotransmitterungleichgewichterkennungssystem (SYS) nach einem der Ansprüche 1 bis 9, wobei die Frequenzanalyseanordnung (FAA) ferner konfiguriert ist, um zu bestimmen, ob der relative Gehalt in dem vordefinierten Frequenzbereich einen vorbestimmten Schwellenwert überschreitet.

11. Neurotransmitterungleichgewichterkennungssystem (SYS) nach einem der Ansprüche 1 bis 10, wobei der vordefinierte Frequenzbereich mindestens den Bereich von 1 Hz bis 5 Hz umfasst.

12. Neurotransmitterungleichgewichterkennungssystem (SYS) nach einem der Ansprüche 1 bis 11, das System (SYS) ferner umfassend eine Analyseeinheit, die konfiguriert ist, um die Anzahl von Augenbewegungsereignissen innerhalb eines vordefinierten Zeitbereichs zu identifizieren.

13. Neurotransmitterungleichgewichterkennungssystem (SYS) nach einem der Ansprüche 1 bis 12, wobei der mindestens eine Augenbewegungssensor (SEN) einen Dehnungsmessstreifensensor umfasst.

14. Neurotransmitterungleichgewichterkennungssystem (SYS) nach einem der Ansprüche 1 bis 13, wobei das System (SYS) konfiguriert ist, um einen Bewertungswert auszugeben, der einen Grad eines positiven Ansprechens auf ein Medikament angibt, wobei der Bewertungswert basiert auf mindestens
- einem ersten vor einer Einnahme des Medikaments aufgezeichneten Augenbewegungssignal (EMS) und
- einem zweiten nach der Einnahme des Medikaments aufgezeichneten Augenbewegungssignal (EMS).

## Revendications

1. Système de détection de déséquilibre de neurotransmetteurs (SYS), ledit système (SYS) comprenant
au moins un capteur de mouvement oculaire (SEN) pour détecter le mouvement d'un œil fermé (EYE),
un agencement d'analyse de fréquence (FAA), et
dans lequel ledit capteur de mouvement oculaire (SEN) est configuré pour émettre au moins un signal de mouvement oculaire (EMS) représentant le mouvement de l'œil fermé (EYE) et pour communiquer ledit au moins un signal de mouvement oculaire (EMS) audit agencement d'analyse de fréquence (FAA),
dans lequel ledit agencement d'analyse de fréquence (FAA) est configuré pour
recevoir ledit au moins un signal de mouvement oculaire (EMS) et
traiter ledit au moins un signal de mouvement oculaire (EMS) par analyse fréquentielle pour déterminer une distribution de fréquences,
émettre un signal d'indication de densité de fréquence (FDIS) corrélé à une densité de fréquence dans une plage de fréquences prédéfinie, et pour
déterminer si ledit signal d'indication de densité de fréquence (FDIS) dépasse un seuil prédéterminé,
identifier une condition anormale lorsque le seuil est dépassé,
dans lequel le déséquilibre des neurotransmetteurs est associé au trouble déficitaire de l'attention avec hyperactivité (TDAH),
dans lequel la plage de fréquences prédéfinie comprend au moins la plage de 1 à 3 Hz, et
dans lequel le seuil prédéterminé est d'au moins 10 % du contenu fréquentiel total.

2. Système de détection de déséquilibre de neurotransmetteurs (SYS) selon la revendication 1, dans lequel l'agencement d'analyse de fréquence (FAA) est en outre configuré pour émettre un signal de contenu fréquentiel (FCS) représentant un contenu fréquentiel dans une plage de fréquences prédéfinie.

3. Système de détection de déséquilibre de neurotransmetteurs (SYS) selon la revendication 1 ou 2, dans lequel le système (SYS) comprend en outre un agencement d'analyse d'oscillation, l'agencement d'analyse d'oscillation, l'agencement d'analyse d'oscillation étant configuré pour
recevoir ledit au moins un signal de mouvement oculaire (EMS), et
traiter ledit au moins un signal de mouvement oculaire (EMS).

4. Système de détection de déséquilibre de neurotransmetteurs (SYS) selon la revendication 3, dans lequel l'agencement d'analyse d'oscillation est configuré pour
traiter ledit au moins un signal de mouvement oculaire (EMS) pour déterminer le taux d'occurrence d'oscillations dépassant un seuil prédéterminé.

5. Système de détection de déséquilibre de neurotransmetteurs (SYS) selon la revendication 3 ou 4, dans lequel l'agencement d'analyse d'oscillation est configuré pour
traiter ledit au moins un signal de mouvement oculaire (EMS) pour déterminer une amplitude représentative de mouvements oculaires associée à une oscillation du signal de mouvement oculaire dépassant un seuil prédéterminé.

6. Système de détection de déséquilibre de neurotransmetteurs (SYS) selon l'une quelconque des revendications 3-5, dans lequel l'agencement d'analyse d'oscillation est configuré pour
traiter ledit au moins un signal de mouvement oculaire (EMS) pour déterminer le taux d'occurrence des passages par zéro du signal de mouvement oculaire (EMS).

7. Système de détection de déséquilibre de neurotransmetteurs (SYS) selon l'une quelconque des revendications 3-6, dans lequel l'agencement d'analyse d'oscillation est configuré pour
traiter ledit au moins un signal de mouvement oculaire (EMS) pour déterminer la somme totale du signal de mouvement oculaire (EMS).

8. Système de détection de déséquilibre de neurotransmetteurs (SYS) selon l'une quelconque des revendications 1-7, dans lequel le système est en outre configuré pour déterminer la valeur maximale de la distribution de fréquences.

9. Système de détection de déséquilibre de neurotransmetteurs (SYS) selon l'une quelconque des revendications 1-8, dans lequel ledit agencement d'analyse de fréquence (FAA) est en outre configuré pour comparer ledit signal de contenu fréquentiel avec une représentation de plage normale et une représentation de plage anormale.

10. Système de détection de déséquilibre de neurotransmetteurs (SYS) selon l'une quelconque des revendications 1-9, dans lequel ledit agencement d'analyse de fréquence (FAA) est en outre configuré pour déterminer si le contenu relatif dans ladite plage de fréquences prédéfinie dépasse un seuil prédéterminé.

11. Système de détection de déséquilibre de neurotransmetteurs (SYS) selon l'une quelconque des revendications 1-10, dans lequel la plage de fréquences prédéfinie comprend au moins la plage de 1 Hz à 5 Hz.

12. Système de détection de déséquilibre de neurotransmetteurs (SYS) selon l'une quelconque des revendications 1-11, dans lequel le système (SYS) comprend en outre une unité d'analyse configurée pour identifier le nombre d'événements de mouvement oculaire dans une plage de temps prédéfinie.

13. Système de détection de déséquilibre de neurotransmetteurs (SYS) selon l'une quelconque des revendications 1-12, dans lequel l'au moins un capteur de mouvement oculaire (SEN) comprend un capteur à jauge de contrainte.

14. Système de détection de déséquilibre de neurotransmetteurs (SYS) selon l'une quelconque des revendications 1-13, dans lequel le système (SYS) est configuré pour émettre une valeur d'évaluation indiquant un degré de réponse positive à un médicament, la valeur d'évaluation étant en fonction d'au moins
- un premier signal de mouvement oculaire (EMS) enregistré avant la prise du médicament et
- un second signal de mouvement oculaire (EMS) enregistré après la prise du médicament.
